# FASCICULE DE BREVET EUROPEEN

(11) **EP 3 645 730 B1**
(45) Date de publication et mention de la délivrance du brevet: **31.07.2024**
(21) Numéro de dépôt: 18750247.1
(22) Date de dépôt: 29.06.2018
(51) Int. Cl.: C12P 7/22, C12N 15/63

(54) **UTILISATION DES POLYKÉTIDE SYNTHASES DE TYPE III COMME PHLOROGLUCINOL SYNTHASES**
VERWENDUNG VON TYP-III-POLYKETID-SYNTHASEN ALS PHLOROGLUCINSYNTHASEN
USE OF TYPE III POLYKETIDE SYNTHASES AS PHLOROGLUCINOL SYNTHASES

(30) Priorité: 30.06.2017 FR 1756108
(43) Date de publication de la demande: 06.05.2020
(73) Titulaire: Compagnie Générale des Etablissements Michelin, 63000 Clermont-Ferrand (FR)
(72) Inventeur: LAFAQUIERE, Vincent, 63040 Clermont Ferrand Cedex 9 (FR); RAMAEN, Odile, 78660 Ablis (FR); LOUIS, Dominique, 91470 Forges les Bains (FR)
(74) Mandataire: M.F.P. Michelin
(86) Numéro de dépôt international: PCT/FR2018/051618
(87) Numéro de publication internationale: WO 2019/002798

(56) Documents cités:
- WO-A2-2012/003461
- US-A1- 2014 315 269
- DATABASE UniProt [online] 3 May 2011 (2011-05-03), "SubName: Full=Uncharacterized protein {ECO:0000313|EMBL:EGB09027.1};", XP002780083, retrieved from EBI accession no. UNIPROT:F0Y6J5 Database accession no. F0Y6J5
- DATABASE UniProt [online] 5 October 2010 (2010-10-05), "SubName: Full=Type III polyketide synthase {ECO:0000313|EMBL:ADK13089.1};", XP002780084, retrieved from EBI accession no. UNIPROT:D9J215 Database accession no. D9J215
- DATABASE EMBL [online] 19 July 2010 (2010-07-19), "Sargassum binderi type III polyketide synthase mRNA, complete cds.", XP002780085, retrieved from EBI accession no. EM_STD:HM245964 Database accession no. HM245964
- HARIYANTI BAHARUM ET AL: "Molecular Cloning, Modeling, and Site-Directed Mutagenesis of Type III Polyketide Synthase from(Phaeophyta)", MARINE BIOTECHNOLOGY, SPRINGER-VERLAG, NE, vol. 13, no. 5, 23 December 2010 (2010-12-23), pages 845 - 856, XP019939493, ISSN: 1436-2236, DOI: 10.1007/S10126-010-9344-5
- L. MESLET-CLADIERE ET AL: "Structure/Function Analysis of a Type III Polyketide Synthase in the Brown Alga Ectocarpus siliculosus Reveals a Biochemical Pathway in Phlorotannin Monomer Biosynthesis", THE PLANT CELL, vol. 25, no. 8, 1 August 2013 (2013-08-01), US, pages 3089 - 3103, XP055466135, ISSN: 1040-4651, DOI: 10.1105/tpc.113.111336
- SEVERIN SASSO ET AL: "Microalgae in the postgenomic era: a blooming reservoir for new natural products", FEMS MICROBIOLOGY REVIEWS, 1 September 2011 (2011-09-01), pages n/a - n/a, XP055020895, ISSN: 0168-6445, DOI: 10.1111/j.1574-6976.2011.00304.x

## Description

### DOMAINE DE L'INVENTION

La présente invention se situe dans les domaines de la biochimie microbienne et plus particulièrement dans le domaine de la synthèse par des enzymes microbiennes du phloroglucinol. Elle concerne l'utilisation des polykétide synthases de type III d'algues comme phloroglucinol synthases, , de préférence d'algues eucaryotes ochrophytes. La présente invention est telle que définie dans les revendications.

### ART ANTERIEUR

Le phloroglucinol est un composé organique aromatique utilisé notamment dans la fabrication de produits pharmaceutiques et d'explosifs.

La synthèse de phloroglucinol est catalysée par des polykétide synthases de type III appelées phloroglucinol synthases. Les phloroglucinol synthases réalisent la condensation de trois molécules de malonyl-CoA pour former une molécule de phloroglucinol selon le schéma réactionnel suivant (Réaction I):

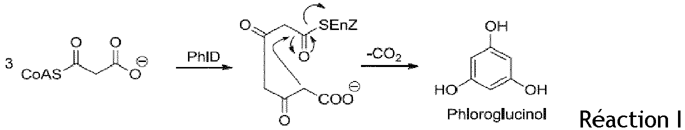

De nombreux oligomères peuvent ensuite être synthétisés à partir du phloroglucinol, tels que les phlorotannins. Les phlorotannins incluent notamment les fucols, les phloretols et les fucophloretols, qui sont des produits dérivés du phloroglucinol composant la paroi des algues brunes. En outre, diverses activités protectrices des algues brunes ont également été attribuées aux phlorotannins.

La base de données Uniprot comprend des séquences pour lesquelles l'annotation mentionne plusieurs activités putatives, donc non vérifiées, dont une activité putative de polykétide synthases de type III (telles que les séquences identifiées par les numéros F0Y6J5 (décrite dans XP-002780083), D9J215 (décrite dans XP-002780084), ou HM245964 (décrite dans XP-002780085)) .

A ce jour, la synthèse du phloroglucinol a été décrite uniquement chez les bactéries Gram-*Pseudomonas fluorescens* (Achkar et al., 2005 ; Zha et al., 2006) et chez l'algue brune *Ectocarpus siliculosus* (Meslet-Cladière et al., 2013). L'enzyme phloroglucinol synthase impliquée dans la synthèse du phloroglucinol a été identifiée chez ces deux espèces. Ce sont les deux seules phloroglucinol synthases identifiées et caractérisées à ce jour.

Chez *Pseudomonas fluorescens,* la phloroglucinol synthase est codée par le gène PHLD (Achkar et al., 2005 ; Zha et al., 2006).

Chez *Ectocarpus siliculosus,* la phloroglucinol synthase est codée par le gène PKS1 (Meslet-Cladière et al., 2013).

L'activité de phloroglucinol synthase de PHLD a pu être démontrée chez *Escherichia coli* exprimant un gène PHLD hétérologue (Achkar et al., 2005). Cette activité a été confirmée in *vitro,* par des tests enzymatiques à petite échelle réalisés avec une PHLD hétérologue exprimée et purifiée à partir de cultures *d'Escherichia coli* recombinantes (Zha et al., 2006).

L'activité de phloroglucinol synthase de **PKS1** a été démontrée *in vitro,* à partir de PKS1 recombinante exprimée et purifiée chez *Escherichia* coli et à partir d'extraits cellulaires d'*E*. *siliculosus* (Meslet-Cladière et al., 2013, WO 2013/045510).

Toutefois, les enzymes PHLD et PKS1 présentent des activités enzymatiques faibles. En outre, la possibilité de synthétiser le phloroglucinol *in vitro* à grande échelle en utilisant ces enzymes n'a jamais été prouvée. Enfin, les phloroglucinol synthases utilisées dans ces travaux ont été produites par des bactéries *E. coli* ou *P. fluorescens.* L'activité de ces enzymes lorsqu'elles sont produites par des eucaryotes, tels que des levures ou des cellules d'insectes ou de mammifères, n'est ainsi pas connue. Or, les systèmes eucaryotes peuvent être avantageux, notamment pour des productions à grande échelle. Ils permettent en effet l'obtention d'enzymes plus proches des enzymes d'origine eucaryotes, notamment parce que celles-ci peuvent être modifiées au niveau post-traductionnel.

Ainsi, il existe toujours le besoin d'identifier de nouvelles phloroglucinol synthases présentant une activité enzymatique *in vitro* ou *in vivo* de synthèse de phloroglucinol élevée, adaptées pour une production à l'échelle industrielle et pouvant être produites par des systèmes eucaryotes.

La caractérisation fonctionnelle exacte d'une polykétide synthase est cependant compliquée par le fait que cette classe d'enzymes rassemble des protéines présentant de grandes similarités de séquences alors qu'elles peuvent catalyser des réactions sensiblement dissemblables et reconnaître des substrats tout à fait différents.

De ce fait, des tentatives d'identification de nouvelles phloroglucinol synthases chez d'autres organismes n'ont pas abouti. En particulier, différentes souches de *Sargassum sp.* sont connues pour être capables de produire du phloroglucinol. Afin d'identifier les enzymes responsables de la synthèse de ce produit, des polykétide synthases de type III putatives ont été identifiées chez S. *binderi.* Ces enzymes candidates ont été testées pour l'activité de phloroglucinol synthase. Ces tests ont révélé qu'aucune de ces polykétide synthases de type III identifiées chez S. *binderi* ne présentait cette activité (Baharum et al., 2011).

Malgré ces difficultés, les présents Inventeurs ont pu identifier des polypeptides ayant une activité phloroglucinol synthase. Ainsi, de nouvelles phloroglucinol synthases ont été identifiées chez des algues ochrophytes, notamment chez S. *binderi* et *A. anophagefferens.* Elles constituent le premier exemple de phloroglucinol synthases chez ce type d'algues. Les Inventeurs démontrent ici que ces nouvelles phloroglucinol synthases présentent une activité de synthèse de phloroglucinol élevée. Elles sont donc adaptées à la production à l'échelle industrielle. De plus, elles sont fonctionnelles lorsqu'elles sont produites en système eucaryote.

### RESUME DE L'INVENTION

Dans le cadre de la présente invention, les Inventeurs ont mis en évidence, de manière tout à fait surprenante, que des organismes vivants, outre *P. fluorescens* et *E. siliculosus,* contiennent dans leur génome un gène codant une polykétide synthase de type III ayant une activité de phloroglucinol synthase fonctionnelle.

La présente invention se rapporte donc à l'utilisation d'au moins un polypeptide choisi parmi les polyketide synthases de type III d'algues, comme phloroglucinol synthase, à l'exclusion des polyketide synthases de type III *d'Ectocarpus siliculosus* choisies parmi PKS1.Es, PKS2.Es, PKS3.Es ; ledit polypeptide comprenant au moins une séquence d'acides aminés ayant au moins 80% d'identité avec une séquence choisie parmi SEQ ID NO: 3 et SEQ ID NO: 1.

La présente invention concerne également un polypeptide isolé à activité de phloroglucinol synthase comprenant au moins une séquence d'acides aminés présentant au moins 80% d'identité avec la séquence SEQ ID NO: 3.

La présente invention concerne également des molécules d'acides nucléiques isolées codant pour le polypeptide isolé selon l'invention.

La présente invention concerne également des molécules d'acides nucléiques isolées comprenant au moins une séquence d'acides nucléiques codant un polypeptide, et comprenant en outre un promoteur contrôlant l'expression de ladite au moins une séquence d'acides nucléiques ; ledit polypeptide étant choisi parmi les polyketide synthases de type III d'algues, à l'exclusion des polyketide synthases de type III *d'Ectocarpus siliculosus* choisies parmi PKS1.Es, PKS2.Es, PKS3.Es ; ledit polypeptide comprenant au moins une séquence d'acides aminés ayant au moins 80% d'identité avec une séquence choisie parmi SEQ ID NO: 3 et SEQ ID NO: 1.

L'invention concerne en outre des vecteurs comprenant au moins une molécule d'acides nucléiques isolée telle que définie ci-dessus, codant pour une telle phloroglucinol synthase.

L'invention concerne également des cellules hôtes comprenant au moins une molécule d'acides nucléiques isolée ou au moins un vecteur selon l'invention, la cellule hôte étant un microorganisme sélectionné parmi les bactéries, les levures, les champignons, et les cyanobactéries.

Il est également ici décrit des méthodes pour produire une phloroglucinol synthase fonctionnelle, qui ne figurent pas dans le texte des revendications.

L'invention concerne également des méthodes pour produire du phloroglucinol, telles que définies dans les revendications.

### DESCRIPTION DES FIGURES

La **Figure 1** montre les alignements des séquences protéiques des enzymes ou candidats d'algues PKS1.Es (PHLD.Es), PHLD.Sbi et PHLD-1.Aa ainsi que celle de la bactérie *Pseudomonas fluorescens* PHLD.Pf, réalisé avec le logiciel Clustal W.
La **Figure 2** présente un exemple de séparation du phloroglucinol / résorcinol sur colonne propyl-pentafluorophenyl (PFP).
La **Figure 3** montre le résultat de la recherche Blast de la partie N-terminale de PHLD.Sbi dans les contigs du génome *d'A. anophagefferens.*
La **Figure 4** montre la localisation de la séquence génomique d'A.anophagefferens codant une séquence peptidique homologue à la partie amino-terminale de PHLD.Sbi (séquence surlignée en gris clair encadrée par 2 flèches noires) située en 5' de la séquence du gène PHLD.Aa (flèche en gris foncé) tel qu'annoté dans les banques de données génomiques.
La **Figure 5** montre la partie amino-terminale de la séquence peptidique reconstituée de l'enzyme PHLD-1.Aa.
La **Figure 6** montre la comparaison entre la structure tridimensionnelle (3D) de l'enzyme PKS1.Es et la structure modélisée de l'enzyme reconstituée PHLD-1.Aa. La partie N-terminale identifiée par homologie avec la séquence de PHLD.Sbi est représentée en gris clair (les flèches indiquent le début de la partie N-terminale de chaque monomère).
La **Figure 7** montre un exemple de la structure d'une unité génique construite pour un candidat donné (PHLD.ii), permettant d'exprimer les gènes PHLD chez la levure *Saccharomyces cerevisiae.*

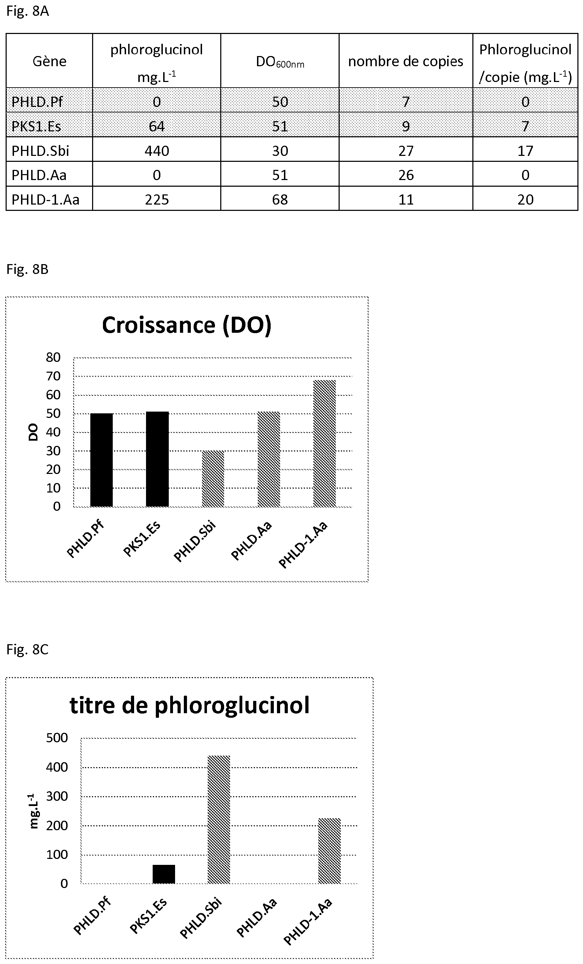

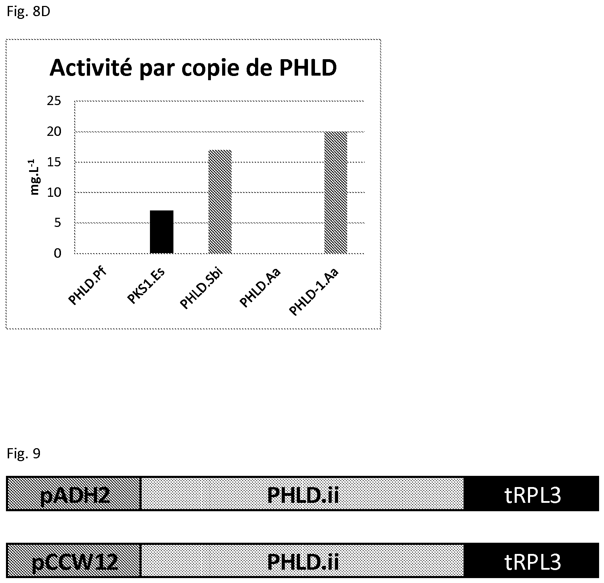

La **Figure 8** montre les niveaux de production de phloroglucinol dans les souches de levure exprimant les différents gènes PHLD.ii et PKS1.Es (plusieurs copies) sous le contrôle du promoteur ADH2, après 48 heures de culture en plaque 24 puits en présence de 20 g.L⁻¹ d'éthanol comme source de carbone à 30°C. (A) Récapitulatif des différentes données mesurées. (B) Densités optiques (DO) des différentes cultures mesurées à 600 nm (DO₆₀₀), indiquant le niveau de croissance de chaque souche. (C) Niveau de production de phloroglucinol (en mg.L⁻¹) mesuré dans le milieu de culture. (D) Niveau de production de phloroglucinol (en mg.L⁻¹) normalisé par rapport au nombre de copies intégrées dans le génome.
La **Figure 9** montre la structure des constructions géniques intégrées dans le génome de la levure au locus JLP1. Le gène codant pour chaque PHLD/PKS1 est sous contrôle du promoteur pADH2 ou du promoteur pCCW12.
La **Figure 10** montre les niveaux de production de phloroglucinol dans les souches de levure exprimant les différents gènes PHLD.ii (1 seule copie) sous le contrôle du promoteur ADH2 après 48 heures de culture en plaque 24 puits en présence de 20 g.L⁻¹ d'éthanol comme source de carbone à 30°C. (A) Récapitulatif des différentes données mesurées. (B) Densités optiques (DO) des différentes cultures mesurées à 600 nm (DO₆₀₀), indiquant le niveau de croissance de chaque souche. (C) Niveau de production de phloroglucinol (en mg.L⁻¹) mesuré dans le milieu de culture.
La **Figure 11** montre les niveaux de production de phloroglucinol dans les souches de levure exprimant les différents gènes PHLD.ii (1 seule copie) sous le contrôle du promoteur CCW12 après 48 heures de culture en plaque 24 puits en présence de 20 g.L⁻¹ de glucose comme source de carbone à 30°C. (A) Récapitulatif des différentes données mesurées. (B) Densités optiques (DO) des différentes cultures mesurées à 600 nm (DO₆₀₀), indiquant le niveau de croissance de chaque souche. (C) Niveau de production de phloroglucinol (en mg.L⁻¹) mesuré dans le milieu de culture.

### DESCRIPTION DETAILLEE DE L'INVENTION

### Définitions

Par « **polykétide synthase de type III** », on entend une enzyme multifonctionnelle ou un complexe enzymatique produisant des polykétides et qui n'utilise pas de domaine de protéine porteuse d'acyle (ou ACP pour « acyl carrier protein »).

Par « **polykétide** », on entend une grande famille de métabolites secondaires chez les bactéries, les mycètes, les plantes et certaines lignées d'animaux qui proviennent de la condensation itérative de sous-unités acétyle ou malonyle par des enzymes polykétide synthases. Les polykétides servent également de matières premières pour la fabrication d'un large éventail de produits naturels et semi-synthétiques.

Par « **phloroglucinol** », on entend un composé organique aromatique **benzène-1,3,5-**triol présentant la formule chimique suivante (Formule I) :

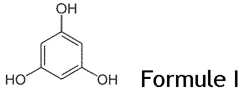

Par « **phloroglucinol synthase** », on entend une enzyme multifonctionnelle ou un complexe enzymatique appartenant à la famille des **polykétide synthases de type III** et catalysant la synthèse du phloroglucinol. Une phloroglucinol synthase catalyse la condensation de trois molécules de malonyl-CoA pour former une molécule de phloroglucinol.

Par « **activité enzymatique** » ou « **activité catalytique** » ou encore « **activité** » d'une enzyme, on entend l'efficacité d'une enzyme à convertir un substrat en produit dans un environnement donné. L'efficacité de l'enzyme prend en compte ici la vitesse de conversion du substrat en produit par l'enzyme et le taux de conversion du substrat en produit par l'enzyme. Par « **taux de conversion du substrat en produit par l'enzyme** » on entend ici le ratio entre la quantité de produit final obtenu par rapport à la quantité initiale de substrat pour une quantité définie d'enzyme. Par exemple, une activité enzymatique au sens de l'invention peut être exprimée en quantité de phloroglucinol produit dans un volume donné (par exemple en g.L⁻¹ ou mg.L⁻¹).

Par « **algue** », on entend un organisme capable de photosynthèse oxygénique et dont le cycle de vie se déroule généralement en milieu aquatique. Les algues comprennent les cyanobactéries et les algues eucaryotes. Par « **cyanobactérie** », on entend un organisme procaryote appartenant au groupe des *Cyanobacteria,* appelées également algues bleues. Par « **algue eucaryote** », on entend un organisme eucaryote appartenant au groupe des algues eucaryotes, comprenant les embranchements des *Glaucophyta* ou *Glaucocystophyta,* des *Rhodophyta,* des *Chlorobionta* ou *Viridiplantae,* des *Cryptophyta,* des *Euglenozoa,* des Cercozoa, des *Haptophyta* ou *Prymnesiophyta,* des *Dinophyta,* et des *Ochrophyta* ou *Heterokontophyta.*

Par « **algue ochrophyte** » ou « **ochrophyte** », on entend une algue appartenant à l'embranchement des *Ochrophyta* appelé également *Heterokontophyta, ochrophytes ou hétérocontophytes.* Les algues ochrophytes sont des algues eucaryotes de couleur brun-doré du règne des *Chromista.*

Par « **algue *Marista*** » ou « ***Marista*** », on entend une algue ochrophyte appartenant à l'infra-embranchement des *Marista.* L'infra-embranchement des *Marista* comprend notamment la classe des *Phaeophyceae* ou Phéophycées et la classe des *Pelagophyceae.*

Par « **algue brune** », on entend un organisme appartenant à la classe des algues brunes appelée également *Phaeophyceae* ou Phéophycées et appartenant à l'embranchement des *Ochrophyta.* Les algues brunes utilisent comme pigment collecteur de lumière de la chlorophylle c combinée à un pigment brun, la fucoxanthine. Leur taille varie de l'échelle microscopique à environ dix mètres de long. Les algues brunes comprennent notamment les algues des genres *Ectocarpus* et *Sargassum.*

Par « **algue pélagophyte** » ou « **pélagophyte** », on entend un organisme appartenant à la classe des algues ***Pelagophyceae,*** appelée également pélagophytes et appartenant à l'embranchement des *Ochrophyta.* Les pélagophytes forment un petit groupe de microalgues eucaryotes.

Par « ***Ectocarpus sp.*** » on entend une algue du genre *Ectocarpus,* de la classe des algues brunes, appartenant à la famille des *Ectocarpaceae.* Le genre *Ectocarpus* comprend notamment l'espèce *Ectocarpus siliculosus.*

Par « ***Sargassum sp.*** » ou « **sargasses** », on entend une algue du genre *Sargassum,* de la classe des algues brunes, appartenant à la famille des *Sargassaceae.* Le genre *Sargassum* comprend notamment l'espèce *Sargassum binderi* (appelée aussi Sbi ci-après).

Par «***Aureococcus sp.***», on entend une algue du genre *Aureococcus,* de la classe des algues pélagophytes. Le genre *Aureococcus* comprend notamment l'espèce *Aureococcus anophagefferens* (appelée aussi Aa ci-après).

*Par « **Pseudomonas sp.*** », on entend une bactérie Gram négative (Gram-) ne formant pas de spores (ou asporulées), en forme de bacille et aérobie obligatoire, du genre *Pseudomonas.* Le genre *Pseudomonas* comprend notamment l'espèce *Pseudomonas fluorescens* (appelée aussi Pf ci-après).

Par « **PHLD.Pf** », on entend indifféremment le gène codant pour la phloroglucinol synthase PHLD de *P. fluorescens,* ainsi que tous les produits de ce gène, y compris les ARN et les polypeptides codés par ce gène.

Par « **PKS1.Es** » ou « **PHLD.Es** », on entend indifféremment le gène codant pour la phloroglucinol synthase PKS1 d'*E. siliculosus,* ainsi que tous les produits de ce gène, y compris les ARN et les polypeptides codés par ce gène.

« **PhlD** » ou « **PHLD** » désigne ici un gène candidat codant pour une enzyme phloroglucinol synthase candidate, ainsi que tous les produits de ce gène, y compris les ARN et les polypeptides codés par ce gène. Selon la nomenclature choisie par les Inventeurs, « **PhlD.ii »** ou « **PHLD.ii** » désigne ici le gène candidat ou le polypeptide candidat issu d'un organisme donné. Les lettres « ii » représentent le genre et l'espèce auxquels ledit organisme appartient.

Par «**molécule d'acides nucléiques** », on entend un polymère de n'importe quelle longueur d'acide désoxyribonucléique (ADN), ou polydésoxyribonucléotides, incluant notamment les ADN complémentaires ou ADNc, les ADN génomiques, les plasmides, les vecteurs, les génomes viraux, l'ADN isolé, les sondes, les amorces et tout mélange de ceux-ci ; ou un polymère de n'importe quelle longueur d'acide ribonucléique (ARN), ou polyribonucléotides, incluant notamment les ARN messagers ou ARNm, ARN antisens ; ou des polyribo-polydésoxyribonucléotides mélangés. Ils englobent des polynucléotides simples ou à double brins, linéaires ou circulaires, naturels ou synthétiques. En outre, un polynucléotide peut comprendre des nucléotides non naturels et peut être interrompu par des composants non nucléotidiques.

Dans le cadre de la présente invention, les termes "acide nucléique", "molécule d'acides nucléiques", "polynucléotide" et "séquence nucléotidique" sont utilisés de manière interchangeable.

Par « **molécule isolée** », on entend une molécule, notamment une protéine, un polypeptide, un peptide, une molécule d'acides nucléiques, un vecteur plasmidique, un vecteur viral ou une cellule hôte, qui est extrait de son environnement naturel (c'est-à-dire séparé d'au moins un autre composant auquel il est naturellement associé).

Par « **polypeptide** », **"protéine"** et **"peptide",** on entend des polymères de résidus d'acides aminés qui comprennent au moins neuf acides aminés liés par des liaisons peptidiques. Le polymère peut être linéaire, ramifié ou cyclique. Le polymère peut comprendre des acides aminés naturels et/ou analogues d'acides aminés et il peut être interrompu par des résidus non-acides aminés. Comme indication générale et sans y être cependant lié dans la présente demande, si le polymère d'acides aminés contient plus de 50 résidus d'acides aminés, il est de préférence appelé un polypeptide ou une protéine, alors que si le polymère est constitué de 50 acides aminés ou moins, il est de préférence appelé "peptide".

Par « **vecteur** », on entend un véhicule, de préférence une molécule d'acide nucléique ou une particule virale, qui contient les éléments nécessaires pour permettre l'administration, la propagation et/ou l'expression d'une ou plusieurs molécule(s) d'acides nucléiques dans une cellule hôte ou un organisme.

D'un point de vue fonctionnel, ce terme englobe des vecteurs pour la maintenance (vecteurs de clonage), des vecteurs pour l'expression dans diverses cellules ou organismes hôtes (vecteurs d'expression), des vecteurs extrachromosomiques (par exemple des plasmides multicopie) ou des vecteurs d'intégration (par exemple conçus pour s'intégrer dans le génome d'une cellule hôte et produire des copies supplémentaires de la molécule d'acides nucléiques qu'il contient lorsque la cellule hôte se réplique). Ce terme englobe également des vecteurs navettes (par exemple, fonctionnant à la fois dans des hôtes procaryotes et/ou eucaryotes) et des vecteurs de transfert (par exemple pour le transfert de molécule(s) d'acides nucléiques dans le génome d'une cellule hôte).

D'un point de vue structurel, les vecteurs selon l'invention peuvent être des sources génétiques naturelles, synthétiques ou artificielles, ou une combinaison d'éléments génétiques naturels et artificiels.

Ainsi, dans le contexte de l'invention, le terme "vecteur" doit être compris de manière large en incluant des vecteurs plasmidiques (ou plasmides) et viraux.

Un **"plasmide"** tel qu'utilisé ici désigne une construction d'ADN réplicable. Habituellement, les vecteurs plasmidiques contiennent des gènes marqueurs de sélection qui permettent aux cellules hôtes portant le plasmide d'être identifiées et/ou sélectionnées de manière positive ou négative en présence du composé correspondant au marqueur de sélection. Une variété de gènes marqueurs de sélection positifs et négatifs sont connus dans la technique. À titre d'illustration, un gène de résistance aux antibiotiques peut être utilisé comme un gène marqueur de sélection positif permettant de sélectionner une cellule hôte en présence de l'antibiotique correspondant.

Le terme **"vecteur viral"** tel qu'utilisé ici se réfère à un vecteur d'acide nucléique qui comprend au moins un élément d'un génome du virus et peut être conditionné dans une particule virale ou une particule virale. Les vecteurs viraux peuvent être compétents pour la réplication ou sélectifs (par exemple, conçus pour se répliquer mieux ou sélectivement dans des cellules hôtes spécifiques), ou peuvent être génétiquement désactivés de manière à être défectueux ou déficients en réplication.

Par « **cellule hôte** », on entend une cellule contenant une molécule d'acides nucléiques selon l'invention. Avantageusement encore, la cellule hôte est capable d'exprimer un polypeptide à activité de phloroglucinol synthase et/ou de produire le vecteur de l'invention. Avantageusement, la cellule hôte est capable de synthétiser du phloroglucinol.

La cellule hôte peut être constituée d'un type unique de cellules ou d'un groupe de différents types de cellules. La cellule hôte peut également être une cellule hybride, c'est-à-dire résultant de la fusion d'au moins deux cellules de types différent.

La cellule hôte peut appartenir à des lignées cellulaires cultivées, à des cellules primaires, à des cellules souches ou à des cellules prolifératives. Dans le cadre de l'invention, la cellule hôte est un microorganisme sélectionné parmi les bactéries, les levures, les champignons, et les cyanobactéries.

Le terme « cellule hôte » comprend plus largement des cellules qui contiennent ou ont contenu la molécule d'acides nucléiques selon l'invention, ainsi que la descendance de telles cellules. La cellule hôte peut par exemple être isolée ou organisée en tissu, en organe ou encore être au sein d'un organisme complet. Dans le cas où la cellule hôte est au sein d'un organisme complet, ledit organisme n'est pas humain.

Il est ainsi clair qu'une « cellule hôte » selon la présente invention est une cellule hôte recombinante, i.e., une cellule hébergeant un matériel génétique exogène. Ainsi, une cellule hôte n'est pas une cellule sauvage qui existe à l'état naturel mais est un outil de biologie moléculaire obtenu par les techniques de manipulations génétiques.

Par « **identité** », on entend une correspondance exacte de séquence entre deux polypeptides ou deux molécules d'acides aminés. Le « **pourcentage d'identité** » entre deux séquences est fonction du nombre de résidus identiques communs aux deux séquences, et prend en compte le nombre d'intervalles qui doivent être introduits pour un alignement optimal et la longueur de chaque intervalle. Divers programmes informatiques et algorithmes mathématiques sont disponibles dans l'état de l'art pour déterminer le pourcentage d'identité entre les séquences d'acides aminés, comme par exemple le programme Blast disponible sur la base NCBI ou ALIGN (Atlas of Protein Séquence and Structure, Dayhoff (ed.), 1981, Suppl. 3 482-489). Des programmes pour déterminer l'homologie entre les séquences nucléotidiques sont également disponibles dans une base de données spécialisée (par exemple Genbank, le Wisconsin Séquence Analysis Package, les programmes BESTFIT, FASTA et GAP). À titre illustratif, "au moins 80% d'identité de séquence", tel qu'utilisé ici, représente 80%, 81%, 82%, 83%, 84%, 85%, 86%, 87%, 88%, 89%, 90%, 91 %, 92%, 93%, 94%, 95%, 96%, 97%, 98%, 99% ou 100%.

Dans la description détaillée qui suit, les modes de réalisation pourront être pris seuls ou combinés de manière appropriée par l'homme du métier.

### Polypeptides isolés et leur utilisation comme phloroglucinol synthases

Les Inventeurs ont identifié, de manière tout à fait surprenante, des gènes codant de nouvelles polykétide synthases de type III dans le génome d'organismes vivants qui n'étaient pas connus pour coder ce type d'enzyme. Les Inventeurs ont notamment mis en évidence, pour la première fois, que ces nouvelles polykétide synthases de type III ont une activité de phloroglucinol synthase.

La présente invention concerne donc un polypeptide isolé à activité de phloroglucinol synthase comprenant au moins une séquence d'acides aminés présentant au moins 80% d'identité avec la séquence SEQ ID NO: 3. De préférence, le polypeptide isolé à activité de phloroglucinol synthase comprend au moins une séquence d'acides aminés présentant au moins 85% d'identité, de préférence encore au moins 90% d'identité, de préférence encore au moins 91% d'identité, de préférence encore au moins 92% d'identité, de manière encore préférée au moins 93% d'identité, de manière encore plus préférée au moins 94% d'identité, de manière toujours plus préférée au moins 95% d'identité, de manière toujours plus préférée au moins 96% d'identité, de manière toujours plus préférée au moins 97% d'identité, de manière toujours plus préférée au moins 98% d'identité, et de manière plus préférentielle encore au moins 99% d'identité avec la séquence SEQ ID NO: 3.

Selon un mode de réalisation, le polypeptide isolé à activité de phloroglucinol synthase comprend au moins une séquence d'acides aminés de séquence SEQ ID NO: 3.

Dans un mode de réalisation préféré, le polypeptide isolé à activité de phloroglucinol synthase possède la séquence d'acides aminés de séquence SEQ ID NO: 3, dont la séquence est indiquée dans le Tableau 2 ci-après.

Avantageusement, le polypeptide isolé à activité de phloroglucinol synthase est le polypeptide isolé PHLD *d'Aureococcus anophagefferens,* en particulier PHLD-1 .Aa, de SEQ ID NO: 3. En effet, les Inventeurs ont identifié de manière tout à fait surprenante une nouvelle protéine, PHLD-1.Aa, qui n'était pas décrite dans les différentes bases de données disponibles pour l'homme du métier. Les Inventeurs ont montré que cette nouvelle protéine PHLD-1.Aa est une polykétide synthase de type III et qu'elle présente une activité de phloroglucinol synthase.

Selon un mode de réalisation, le polypeptide à activité de phloroglucinol synthase est isolé à partir de souches *d'Aureococcus anophagefferens* en culture.

Selon un mode de réalisation, le polypeptide à activité de phloroglucinol synthase est isolé à partir d'une cellule hôte hétérologue exprimant ledit polypeptide, ladite cellule hôte étant telle que définie précédemment et que décrite ci-dessous dans la section « Cellules hôtes ».

Selon un mode de réalisation, le polypeptide isolé à activité de phloroglucinol synthase est synthétisé *in vitro* par des techniques de synthèse protéique que l'homme du métier sait parfaitement définir.

Selon un mode de réalisation, le polypeptide isolé à activité de phloroglucinol synthase est recombinant.

La présente invention concerne également l'utilisation d'au moins un polypeptide choisi parmi les polyketide synthases de type III d'algues, comme phloroglucinol synthase, à l'exclusion des polyketide synthases de type III *d'Ectocarpus siliculosus* choisies parmi PKS1.Es, PKS2.Es, PKS3.Es; ledit polypeptide comprenant au moins une séquence d'acides aminés ayant au moins 80% d'identité avec une séquence choisie parmi SEQ ID NO: 3 et SEQ ID NO: 1, dont les séquences sont indiquées dans le Tableau 2 ci-après.

Dans un mode de réalisation préféré, ledit polypeptide est choisi parmi les polyketide synthases de type III d'algues ochrophytes, de préférence d'algues *Marista,* de préférence encore d'algues appartenant à la classe des algues brunes ou à la classe des algues pélagophytes.

Selon un mode de réalisation, ledit polypeptide isolé est choisi parmi les polyketide synthases de type III des algues brunes, à l'exclusion des polyketide synthases de type III *d'Ectocarpus siliculosus* choisies parmi PKS1.Es, PKS2.Es, PKS3.Es, de préférence à l'exclusion de PKS1.Es. Avantageusement, ledit polypeptide isolé est choisi parmi les polyketide synthases de type III d'algues *Sargassum sp.,* en particulier *Sargassum binderi.*

Selon un autre mode de réalisation avantageux, ledit polypeptide est choisi parmi les polyketide synthases de type III d'algues pélagophytes. Avantageusement, ledit polypeptide isolé est choisi parmi les polyketide synthases de type III d'algues *Aureococcus sp.,* en particulier *Aureococcus anophagefferens.*

Ledit polypeptide comprend au moins une séquence d'acides aminés ayant de préférence au moins 80% d'identité, de préférence encore au moins 85% d'identité, de manière encore préférée au moins 90% d'identité, de manière encore plus préférée au moins 91% d'identité, de manière toujours plus préférée au moins 92% d'identité, de manière plus préférentielle encore au moins 93% d'identité, de manière encore plus préférentielle au moins 94% d'identité, de manière davantage préférée au moins 95% d'identité, de manière encore préférée au moins 96% d'identité, de manière encore plus préférée au moins 97% d'identité, de manière toujours plus préférée au moins 98% d'identité, et de manière plus préférentielle encore au moins 99% d'identité, avec une séquence choisie parmi SEQ ID NO: 1 et SEQ ID NO: 3.

Selon un mode de réalisation particulièrement avantageux, ledit polypeptide comprend au moins une séquence d'acides aminés choisie parmi SEQ ID NO: 1 et SEQ ID NO: 3.

Dans un mode de réalisation préféré, le polypeptide isolé à activité de phloroglucinol synthase possède une séquence d'acides aminés choisie parmi SEQ ID NO: 1 et SEQ ID NO: 3.

Avantageusement, le polypeptide isolé à activité de phloroglucinol synthase est choisi parmi le polypeptide isolé à activité de phloroglucinol synthase PHLD.Sbi de *Sargassum binderi* et le polypeptide isolé à activité de phloroglucinol synthase PHLD-1.Aa *d'Aureococcus anophagefferens.*

### Molécules d'acides nucléiques isolées

La présente invention concerne des molécules d'acides nucléiques isolées comprenant au moins une séquence d'acides nucléiques codant un polypeptide choisi parmi les polykétide synthases de type III d'algues (notamment les polyketide synthases de type III d'algues eucaryotes ochrophytes), et comprenant en outre un promoteur contrôlant l'expression de ladite au moins une séquence d'acides nucléiques, ledit polypeptide étant tel que défini ci-dessus. Ledit polypeptide est donc un polypeptide choisi parmi les polyketide synthases de type III d'algues, à l'exclusion des polyketide synthases de type III *d'Ectocarpus siliculosus* choisies parmi PKS1.Es, PKS2.Es, PKS3.Es, ledit polypeptide comprenant au moins une séquence d'acides aminés ayant au moins 80% d'identité avec une séquence choisie parmi SEQ ID NO: 3 et SEQ ID NO: 1.

De manière avantageuse, le promoteur est un promoteur exogène, en particulier un promoteur de levure, de préférence un promoteur choisi parmi ADH2 (pADH2) et CCW12 (pCCW12), de préférence encore un promoteur choisi parmi ADH2 (pADH2) de *Saccharomyces cerevisiae* et CCW12 de *S*. *cerevisiae,* de préférence encore un promoteur choisi parmi ADH2 (pADH2) de SEQ ID NO: 7 et CCW12 de SEQ ID NO: 8.

Selon un mode de réalisation, la molécule d'acides nucléiques isolée comprend en outre un terminateur de transcription d'au moins une séquence d'acides nucléiques codant un polypeptide tel que défini ci-dessus. Ainsi, selon un mode de réalisation, la présente invention concerne une molécule d'acides nucléiques isolée telle que définie ci-dessus, comprenant au moins une séquence d'acides nucléiques codant un polypeptide choisi parmi les polykétide synthases de type III tel que défini ci-dessus, et comprenant en outre un terminateur contrôlant l'expression de ladite au moins une séquence d'acides nucléiques.

De manière avantageuse, le terminateur est un terminateur exogène, en particulier un terminateur de levure, de préférence le terminateur RPL3 (tRPL3), de préférence encore le terminateur RPL3 de *S*. *cerevisiae,* de préférence encore le terminateur RPL3 de SEQ ID NO: 9. Selon un mode de réalisation préféré, la molécule d'acides nucléiques isolée comprend à la fois un promoteur et un terminateur qui sont tels que définis ci-dessus. Ainsi, selon un mode de réalisation, la présente invention concerne une molécule d'acides nucléiques isolée comprenant au moins une séquence d'acides nucléiques codant un polypeptide choisi parmi les polykétide synthases de type III tel que défini ci-dessus, et comprenant en outre un promoteur et un terminateur contrôlant l'expression de ladite au moins une séquence d'acides nucléiques.

Selon un mode de réalisation, la molécule d'acides nucléiques comprend en outre une séquence d'export. Avantageusement, cette séquence d'export permet la sécrétion ou l'excrétion du ou des polypeptides codés par ladite molécule d'acides nucléiques, dans le milieu cellulaire.

Selon un mode de réalisation, la molécule d'acides nucléiques est isolée à partir de souches homologues en culture, de préférence choisies parmi *Sargassum binderi et Aureococcus anophagefferens.*

Selon un mode de réalisation, la molécule d'acides nucléiques est isolée à partir d'un vecteur ou d'une cellule hôte hétérologue comprenant ladite molécule, ledit vecteur ou ladite cellule hôte étant tel(le) que défini(e) ci-après dans la section « Vecteurs » ou « Cellules hôtes ».

Selon un mode de réalisation, la molécule d'acides nucléiques isolée est synthétisée *in vitro* par des techniques de synthèse nucléique que l'homme du métier sait parfaitement définir. Selon un mode de réalisation, la molécule d'acides nucléiques isolée est recombinante.

### Molécules d'acides nucléiques isolées de séquence PHLD-1.Aa

Selon un autre aspect, la présente invention concerne des molécules d'acides nucléiques isolées codant pour un polypeptide isolé à activité de phloroglucinol synthase comprenant au moins une séquence d'acides aminés présentant au moins 80% d'identité avec la séquence SEQ ID NO: 3, de préférence au moins 85% d'identité, de préférence encore au moins 90% d'identité, de préférence encore au moins 91% d'identité, de préférence encore au moins 92% d'identité, de manière encore préférée au moins 93% d'identité, de manière encore plus préférée au moins 94% d'identité, de manière toujours plus préférée au moins 95% d'identité, de manière toujours plus préférée au moins 96% d'identité, de manière toujours plus préférée au moins 97% d'identité, de manière toujours plus préférée au moins 98% d'identité, et de manière plus préférentielle encore au moins 99% d'identité avec la séquence SEQ ID NO: 3.

Avantageusement, la molécule d'acides nucléiques isolées comprend la séquence PHLD-1.Aa.

En accord avec les définitions précédentes, par « **PHLD-1.Aa** », on entend indifféremment le gène codant pour la phloroglucinol synthase PHLD-1.Aa *d'Aureococcus anophagefferens,* ainsi que tous les produits de ce gène, y compris les ARN et les polypeptides codés par ce gène.

Selon un mode de réalisation préféré, la molécule d'acides nucléiques isolée comprend au moins une séquence d'acides nucléiques présentant au moins 80% d'identité avec la séquence SEQ ID NO: 4. De préférence, ladite molécule d'acides nucléiques isolée comprend au moins une séquence d'acides nucléique présentant au moins 85% d'identité, de préférence encore au moins 90% d'identité, de préférence encore au moins 91% d'identité, de préférence encore au moins 92% d'identité, de manière encore préférée au moins 93% d'identité, de manière encore plus préférée au moins 94% d'identité, de manière toujours plus préférée au moins 95% d'identité, de manière toujours plus préférée au moins 96% d'identité, de manière toujours plus préférée au moins 97% d'identité, de manière toujours plus préférée au moins 98% d'identité, et de manière plus préférentielle encore au moins 99% d'identité avec la séquence SEQ ID NO: 4.

Selon un mode de réalisation, la molécule d'acides nucléiques isolée comprend au moins une séquence d'acides aminés de séquence SEQ ID NO: 4.

Dans un mode de réalisation préféré, ladite molécule d'acides nucléiques isolée a pour séquence PHLD-1.Aa. De préférence, ladite séquence PHLD-1.Aa est la séquence SEQ ID NO: 4, dont la séquence est indiquée dans le tableau 2 ci-après.

Selon un mode de réalisation, ladite molécule d'acides nucléiques isolée est isolée à partir de souches *d'Aureococcus anophagefferens* en culture.

Selon un mode de réalisation, ladite molécule d'acides nucléiques isolée est isolée à partir d'un vecteur ou d'une cellule hôte hétérologue comprenant ladite molécule, ledit vecteur ou ladite cellule hôte étant tel(le) que défini(e) ci-après dans la section « Vecteurs » ou « Cellules hôtes ».

Selon un mode de réalisation, ladite molécule d'acides nucléiques isolée est synthétisée *in vitro* par des techniques de synthèse nucléique que l'homme du métier sait parfaitement définir.

Selon un mode de réalisation, ladite molécule d'acides nucléiques isolée est recombinante.

Selon divers modes de réalisation, ladite molécule d'acides nucléiques isolée est conforme à la description ci-dessus dans la Section « Molécules d'acides nucléiques isolées ».

En particulier, ladite molécule d'acides nucléiques isolée peut comprendre un promoteur et/ou un terminateur de transcription et/ou une séquence d'export (voir ci-dessus pour plus de détails).

### Vecteurs

La présente invention concerne des vecteurs comprenant au moins une molécule d'acides nucléiques telle que définie ci-dessus.

Les vecteurs qui sont appropriés dans le cadre de la présente invention comprennent, sans limitation, des vecteurs bactériophages, plasmides ou cosmides pour l'expression dans des cellules hôtes procaryotes telles que des bactéries (par exemple *E. coli,* ou des bactéries du genre *Pseudomonas*); des vecteurs pour l'expression chez la levure (par exemple *Saccharomyces cerevisiae, Schyzosaccharomyces pombe, Pichia pastoris*); des vecteurs de baculovirus pour l'expression dans des systèmes de cellules d'insectes (par exemple, des cellules Sf9); des vecteurs viraux et plasmidiques pour l'expression dans des systèmes de cellules végétales (par exemple le plasmide Ti, le virus de la mosaïque du chou-fleur, CaMV, le virus de la mosaïque du tabac TMV); ainsi que des vecteurs viraux et plasmidiques pour l'expression dans des cellules ou des organismes eucaryotes supérieurs.

Ces vecteurs sont généralement disponibles dans le commerce (par exemple, auprès de fournisseurs tels Invitrogen, Stratagene, Amersham Biosciences, Promega, etc.), disponibles auprès d'institutions de dépôt telles que American Type Culture Collection (ATCC, Rockville, Md.), ou ont fait l'objet de nombreuses publications décrivant leur séquence, leur structure et leurs méthodes de production, de sorte que l'homme du métier peut les appliquer sans difficultés.

Des exemples représentatifs de vecteurs plasmidiques appropriés comprennent, sans limitation, pREP4, pCEP4 (Invitrogen), pCI (Promega), pVAX (Invitrogen) et pgWiz (Gene Therapy System Inc).

Ainsi, avantageusement, le vecteur est un plasmide.

### Cellules hôtes

Dans un autre aspect, la présente invention concerne des cellules hôtes comprenant au moins une molécule d'acides nucléiques ou au moins un vecteur tel que défini ci-dessus, la cellule hôte étant un microorganisme sélectionné parmi les bactéries, les levures, les champignonset les cyanobactéries.

La cellule hôte est de préférence une levure, ladite levure étant en particulier sélectionnée parmi les genres *Saccharomyces, Candida, Ashbya, Dekkera, Pichia* (*Hansenula*), *Debaryomyces, Clavispora, Lodderomyces, Yarrowia, Zigosaccharomyces, Schizosaccharomyces, Torulaspora, Kluyveromyces, Brettanomycces, Cryptococcus* et *Malassezia.*

De manière encore plus particulière, la levure est sélectionnée parmi les espèces *Saccharomyces cerevisiae, Saccharomyces boulardii, Saccharomyces douglasii, Saccharomyces bayanus, Zigosaccharomyces bailii, Schizosaccharomyces pombe, Dekkera brucelensis, Dekkera intermedia, Brettanomycces custersii, Brettanomycces intermedius, Kluyveromyces themotolerens, Torulaspora globosa* et *Torulaspora glabrata.*

Encore plus particulièrement, la levure est du genre *Saccharomyces,* de préférence de l'espèce *Saccharomyces cerevisiae.*

Selon un mode de réalisation, la cellule hôte comprend au moins une copie de la molécule d'acides nucléiques telle que définie ci-dessus intégrée dans son génome.

Selon un mode de réalisation, la cellule hôte comprend une copie unique de la molécule d'acides nucléiques telle que définie ci-dessus intégrée dans son génome.

Lorsque la cellule hôte est une cellule de levure, la ou les copies de la molécule d'acides nucléiques peut être intégrée à différents loci, préférentiellement au locus URA3, au locus JLP1, au locus LEU2, ou au locus TRP1 du génome de ladite cellule de levure. Lorsque la cellule hôte est une cellule de levure et que plusieurs copies de la molécule d'acides nucléiques sont intégrées, les différentes copies peuvent être intégrées au même locus, ou encore à différents loci, préférentiellement à l'une quelconque des combinaisons des loci URA3, JLP1, LEU2, et/ou TRP1.

Avantageusement, les codons utilisés dans la molécule d'acides nucléiques ont été adaptés pour une expression optimale dans la cellule hôte sélectionnée.

Une expression optimale peut notamment être obtenue lorsque les codons choisis sont ceux utilisés de manière préférentielle par l'organisme d'origine de la cellule hôte. Les codons utilisés de manière préférentielle sont connus pour la plupart des organismes couramment utilisés dans le domaine. L'homme du métier pourra aisément déterminer les codons les plus avantageux à utiliser en fonction de la cellule hôte choisie.

A cet effet, l'homme du métier sait quelle technique utiliser pour modifier les codons de la molécule d'acide nucléique. Les codons peuvent être par exemple modifiés par mutagénèse dirigée *in vitro* à partir d'un échantillon de la molécule d'acides nucléiques dont les codons sont à adapter, à l'aide d'une amplification par réaction en chaîne par polymérase (PCR). Alternativement, la molécule d'acides nucléiques peut être synthétisée *in vitro* directement avec les codons optimisés.

Les cellules hôtes peuvent être cultivées dans des bioréacteurs aérobie ou anaérobie, à petite et grande échelle, dans des flacons ou des boites de Pétri. La culture peut être effectuée à une température, un pH, un milieu de culture et une teneur en oxygène appropriés pour une cellule hôte donnée.

### Méthodes

### Méthode pour produire un polypeptide à activité de phloroglucinol synthase

Il est également ici décrit une méthode pour produire un polypeptide à activité de phloroglucinol synthase tel que défini ci-dessus, ladite méthode ne figurant pas dans le texte des revendications.

Selon un mode de réalisation, la méthode pour produire un polypeptide à activité de phloroglucinol synthase tel que défini ci-dessus comprend au moins les étapes consistant en :
(i) l'introduction d'une molécule d'acides nucléiques ou d'un vecteur tels que décrits ci-dessus dans une cellule hôte appropriée conforme à la description qui précède ; et
(ii) la culture *in vitro* de ladite cellule hôte obtenue à l'étape (i) dans des conditions permettant la croissance de ladite cellule hôte et/ou l'expression de ladite molécule d'acides nucléiques, de manière à produire ledit polypeptide.

Selon un autre mode de réalisation, la méthode pour produire un polypeptide à activité de phloroglucinol synthase tel que défini ci-dessus comprend au moins l'étape consistant en :
(i) la culture *in vitro* d'une cellule hôte exprimant ledit polypeptide, par exemple une cellule hôte telle que décrite ci-dessus, dans des conditions permettant la croissance de ladite cellule hôte et/ou l'expression de la molécule d'acides nucléiques contenue dans ladite cellule hôte, de manière à produire ledit polypeptide.

De manière avantageuse, la méthode pour produire un polypeptide à activité de phloroglucinol synthase comprend au moins une étape additionnelle choisie parmi les étapes consistant en :
(α) la récupération des cellules exprimant ledit polypeptide, obtenues après l'étape de culture ; et
(B) la purification du polypeptide à partir des cellules récupérées à l'étape (α).

### Méthode pour produire du phloroglucinol

La présente invention concerne également une méthode pour produire du phloroglucinol. Selon un mode de réalisation M1 qui ne figure pas dans le texte des revendications, la méthode pour produire du phloroglucinol comprend au moins les étapes consistant en :
(i) l'obtention de cellules par la mise en oeuvre de l'une des méthodes décrites ci-dessus ;
(ii) la mise en contact des cellules obtenues à l'étape (i) avec un substrat approprié ;
(iii) l'incubation du mélange issu de l'étape (ii) dans des conditions adaptées pour produire du phloroglucinol;
(iv) optionnellement la récupération du milieu réactionnel comprenant le phloroglucinol, obtenu après l'étape (iii) ; et
(v) optionnellement, la purification du phloroglucinol à partir du milieu réactionnel de l'étape (iv).

Selon un autre mode de réalisation M2, la méthode pour produire du phloroglucinol comprend les étapes consistant en :
(i) la mise en contact d'une cellule hôte exprimant le polypeptide à activité de phloroglucinol synthase tel que défini ci-dessus, par exemple une cellule hôte telle que définie ci-dessus, avec un substrat approprié ;
(ii) la culture *in vitro* de la cellule hôte de l'étape (i) dans des conditions permettant la croissance de ladite cellule hôte et/ou l'expression de la molécule d'acides nucléiques contenue dans ladite cellule hôte, de manière à produire du phloroglucinol ;
(iii) optionnellement la récupération du milieu de culture comprenant le phloroglucinol, obtenu après l'étape (ii) ; et
(iv) optionnellement, la purification du phloroglucinol à partir du milieu de culture de l'étape (iii).

Aux fins des méthodes M1 et M2, le substrat est une source de carbone. Avantageusement, la source de carbone est une source de carbone pure ou un coproduit industriel (tel que la mélasse ou les égouts pauvres, par exemple issus de l'industrie sucrière). De préférence, le substrat dans la source de carbone pure ou le coproduit industriel est un sucre simple, tel que le glucose (ou dextrose), le fructose, le galactose, le mannose, le saccharose, le lactose, ou le maltose ; un sucre complexe, tel qu'un monosaccharide, un disaccharide ou trisaccharides, ou encore un polysaccharide comme l'amidon ; un alcool, tel que l'éthanol ; un acide ; un acide gras et leur dérivé d'ester ; ou un mélange de sucres, d'alcools, d'acides et/ou d'acides gras ou leurs dérivés d'ester.

De préférence, le substrat est le glucose ou le saccharose. Alternativement, le substrat est l'éthanol.

Selon un autre mode de réalisation M3 qui ne figure pas dans le texte des revendications, la méthode pour produire du phloroglucinol comprend au moins les étapes consistant en :
(i) la mise en contact d'au moins un polypeptide obtenu à l'étape (B) de la méthode telle que décrite ci-dessus, avec un substrat approprié ;
(ii) l'incubation du mélange issu de l'étape (i) dans des conditions adaptées pour produire du phloroglucinol ;
(iii) optionnellement la récupération du milieu réactionnel comprenant le phloroglucinol, obtenu après l'étape (ii) ; et
(iv) optionnellement, la purification du phloroglucinol à partir du milieu réactionnel de l'étape (iii).

Selon un autre mode de réalisation M4, la méthode pour produire du phloroglucinol comprend au moins les étapes consistant en :
(i) la mise en contact d'au moins un polypeptide tel que défini ci-dessus avec un substrat approprié ;
(ii) l'incubation du mélange issu de l'étape (i) dans des conditions adaptées pour produire du phloroglucinol ;
(iii) optionnellement la récupération du milieu réactionnel comprenant le phloroglucinol, obtenu après l'étape (ii) ; et
(iv) optionnellement, la purification du phloroglucinol à partir du milieu réactionnel de l'étape (iii).

Aux fins des méthodes M3 et M4, le substrat est un thioester. Avantageusement, le substrat est un acyl-Coenzyme A (ou acyl-CoA) tel que le malonyl-CoA, l'acétyl-CoA, l'hexanoyl-CoA, le decanoyl-CoA, le lauroyl-CoA et le palmitoyl-CoA, ou un mélange de ceux-ci. De préférence, le substrat est le malonyl-CoA.

Selon un mode de réalisation préféré, la purification du phloroglucinol est réalisée par extraction liquide-liquide.

Les exemples qui suivent visent à illustrer la présente invention sans aucune limitation.

Les enzymes respectivement codées par le gène **PHLD** de *Pseudomonas fluorescens* (Zha et al., 2006), par le gène **PKS1** *d'Ectocarpus siliculosus* (Meslet-Cladière et al., 2013) y sont utilisées comme contrôles.

### EXEMPLES

### Exemple 1 : Identification de nouvelles phloroglucinol synthases candidates

Jusqu'à la présente invention, seules deux phloroglucinol synthases avaient été identifiées et caractérisées :
- l'enzyme codée par le gène **PHLD** de *Pseudomonas fluorescens* (Zha et al., 2006), et
- l'enzyme codée par le gène **PKS1** *d'Ectocarpus siliculosus* (Meslet-Cladière et al., 2013).

Les inventeurs ont à présent découvert et caractérisé de nouvelles phloroglucinol synthases en utilisant des analyses génétiques et fonctionnelles.

Comme indiqué dans l'introduction ci-dessus, la caractérisation fonctionnelle exacte d'une polykétide synthase est complexe car cette classe d'enzymes rassemble des protéines présentant de grandes similarités de séquences alors qu'elles peuvent catalyser des réactions sensiblement dissemblables et reconnaître des substrats tout à fait différents.

### 1.1. Sélection de nouvelles enzymes candidates

Afin d'identifier de nouvelles phloroglucinol synthases, les Inventeurs ont identifié des séquences codant pour des polykétide synthases de type III putatives. Les séquences de ces polykétide synthases de type III putatives ainsi identifiées par les Inventeurs ont été analysées et alignées entre elles en utilisant notamment comme base l'alignement des polykétide synthases de type III publié par Meslet-Cladière *et al.* 2013.

L'analyse de cet alignement de séquence a conduit à la sélection d'un groupe d'enzymes candidates, dont les séquences protéiques sont proches de celle du produit du gène PKS1.Es de l'algue *Ectocarpus siliculosus* (Tableaux 1 et 2) :

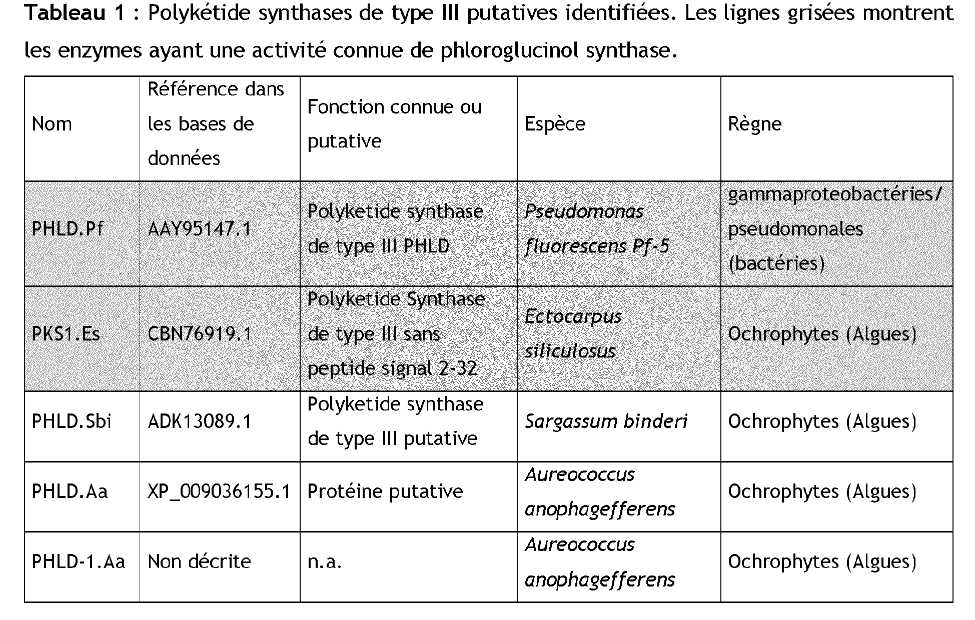

**Tableau 2 : Séquences protéiques ou nucléiques des polykétide synthases de type III putatives identifiées.**

| **SEQ ID NO:** | **Nom** | **Séquence protéique ou nucléique** |
|---|---|---|
| SEQ ID NO: 1 | PHLD.Sbi | MSSAAVAMLADPTVQIALACIVLSLIVVFRSYRKGKDEQTVYPVIAGMAI GNPQYRCTQDQALTVAQKCPGVESVKPVLERIYGNSRIGSRYFAVPDF TPNQAAKGDPMFFPADGSFEVPVDTRLDKFKEKAVPLVSDVARRAIKE AGIDVSDVSKLVVVSSTGFLGPGLDCELIKNLGLTRSVDRTLIGFMGCA AAMNGFRNANDFVTANPGKYALMICVELSSVHTTFDDNINDAILHAIFA DGCAAAVLKGVRKEAPKGTLAIVDNHAWLMEGTEDGITLAIKPNGITC TLSKFLPQYIAKNIAFFADGFLKKHNLGRDDVDFWCVHPGGRRIIEEAQ NGLGLTEAQTADSWAVLAEYGNMLSPSVMFVLSRVFKRHNAALAQGK PGYQTGMAFSFSPGVGAEGILLRQI |
| SEQ ID NO: 2 | PHLD.Aa | MPVEKRLDMFREKSVPLVTKVCKDAMADAGIDVEQIGKLVVVSSTGFLGPG LDAELIKTLGLWRGVDRSLIGFMGCAAAMNGFRVANDFAMSHPGKMAL MVCVEISSVHTTFDDNVNDAILHAIFADGCAAAVISGEKPGSAAAKGKFGIV DTHGWLMEGTEDGITLSINENGISCILSKYLPQYIAKNMAGYVDSFLGMHG LQKTDMDFWAIHPGGRRIIEEAQNGLGLSEEQAKYSWTVLSQYGNMLSPS VMFVLELILNDHKKALAKGERGLKQGIAFSFSPGVGAEGILINVM |
| SEQ ID NO: 3 | PHLD-1.Aa | mskkdekiipvimgmatgnppyrasqqqalaiaescpecnsikpvlariygnsridy rfmavpdftpeqklegdenffdkdlmfkMPVEKRLDMFREKSVPLVTKVCK |
| | | DAMADAGIDVEQIGKLVVVSSTGFLGPGLDAELIKTLGLWRGVDRSLIG FMGCAAAMNGFRVANDFAMSHPGKMALMVCVEISSVHTTFDDNVND AILHAIFADGCAAAVISGEKPGSAAAKGKFGIVDTHGWLMEGTEDGITL SINENGISCILSKYLPQYIAKNMAGYVDSFLGMHGLQKTDMDFWAIHPG GRRIIEEAQNGLGLSEEQAKYSWTVLSQYGNMLSPSVMFVLELILNDHK KALAKGERGLKQGIAFSFSPGVGAEGILINVM |
| SEQ ID NO: 4 | PHLD-1.Aa | ATGTCGAAGAAGGATGAGAAGATCATCCCTGTCATCATGGGTATG GCCACAGGTAATCCGCCATACAGGGCGAGTCAACAACAAGCTCTA GCAATTGCGGAGTCCTGCCCAGAATGTAATAGCATTAAACCGGTA TTGGCAAGAATTTATGGTAATAGCCGTATCGACTACAGGTTTATG GCAGTACCGGATTTCACACCCGAACAGAAGCTTGAAGGTGACGAA AACTTCTTTGATAAAGATTTAATGTTTAAGATGCCAGTGGAGAAG AGATTGGACATGTTTAGGGAGAAGAGTGTGCCATTAGTCACGAAG GTCTGTAAGGATGCCATGGCAGACGCTGGTATAGACGTTGAACAG ATTGGCAAACTTGTCGTGGTATCATCAACTGGCTTCTTAGGCCCT GGACTAGATGCGGAGTTAATTAAGACACTTGGACTTTGGCGTGGT GTTGACCGGTCTCTGATTGGATTCATGGGTTGTGCGGCGGCCAT GAACGGTTTTCGGGTTGCTAATGATTTCGCCATGTCCCACCCGGG AAAGATGGCCCTAATGGTTTGCGTCGAAATATCCTCTGTGCATAC CACATTCGATGATAACGTAAATGATGCAATACTTCATGCGATCTTC GCCGATGGATGTGCAGCCGCAGTAATATCGGGAGAGAAGCCAGG TTCTGCGGCAGCGAAAGGCAAATTCGGAATCGTCGATACGCATGG TTGGCTCATGGAAGGCACAGAAGATGGGATAACCCTGTCTATCAA TGAAAATGGTATATCATGCATCTTGAGTAAGTATCTACCACAGTAT ATTGCAAAGAACATGGCAGGTTACGTAGATAGTTTCCTAGGGATG CATGGATTACAGAAGACAGATATGGATTTCTGGGCTATTCACCCC GGCGGCCGCAGAATTATAGAGGAAGCGCAGAACGGTTTGGGTTT ATCCGAGGAGCAGGCGAAGTATTCTTGGACTGTTCTTAGTCAGTA TGGTAATATGTTGTCACCTAGCGTGATGTTTGTGTTAGAACTGAT CCTTAATGACCACAAGAAGGCACTGGCAAAGGGAGAGCGCGGTTT AAAGCAAGGTATCGCATTTAGCTTCTCACCGGGTGTTGGGGCCGA GGGCATCCTCATTAATGTTATGTAA |

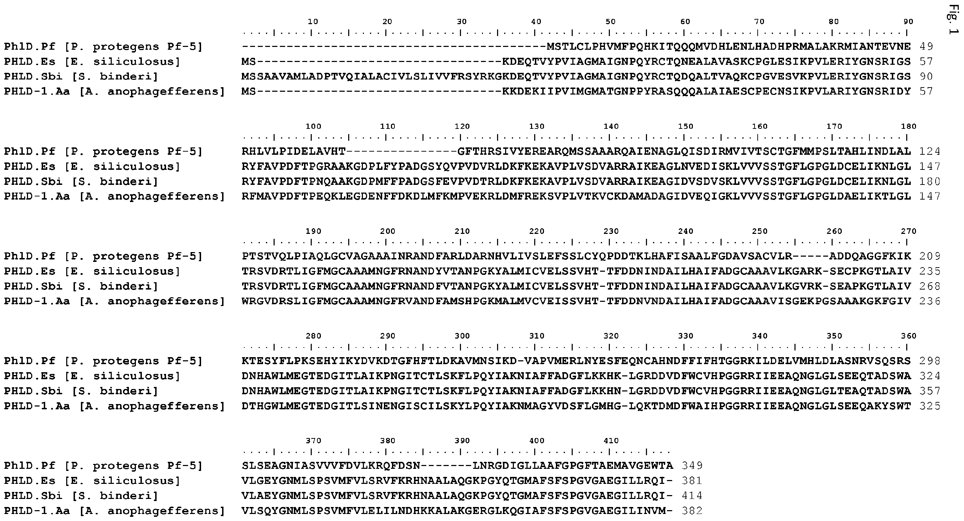

La Figure 1 présente l'alignement des séquences protéiques des enzymes ou candidats chez les algues PKS1 .Es, PHLD.Sbi et PHLD-1 .Aa ainsi que celle de la bactérie *Pseudomonas fluorescens* PHLD.Pf. L'alignement des deux enzymes candidates sélectionnées et de PKS1 .Es avec l'enzyme PHLD.Pf a été réalisé en employant le logiciel Clustal W.

Le Tableau 3 présente la matrice des identités de séquence existant entre ces différentes enzymes.

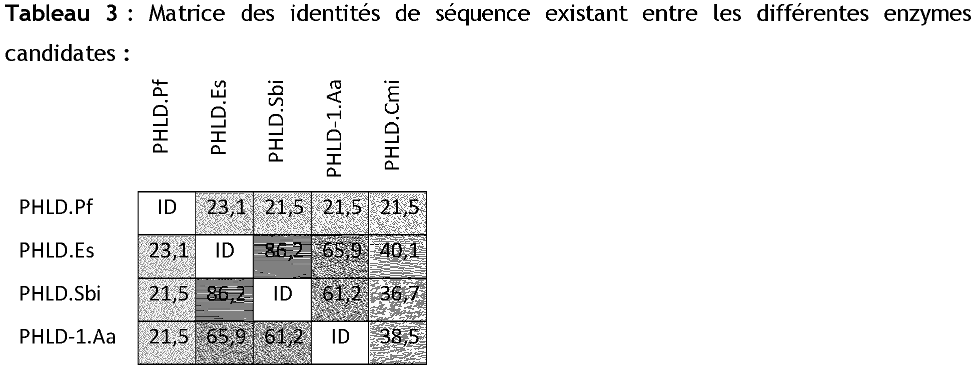

Les résultats montrent que la distance phylogénétique existant entre l'enzyme d'algue PKS1.ES et l'enzyme bactérienne PHLD.Pf est importante puisque moins de 25 % d'identité sont observés entre ces deux enzymes. Il n'est donc pas possible d'affecter *a priori* une fonction phloroglucinol synthase aux polykétide synthases putatives étudiées à cause de cette forte divergence de séquences.

### 1.2. Mesure des activités phloroglucinol synthases chez la levure

Afin d'identifier les phloroglucinol synthases parmi les candidats identifiés ci-dessus, une méthode d'extraction et de dosage du phloroglucinol a été mise au point comme détaillé ci-dessous.

### 1.2.1. Méthode d'extraction du phloroglucinol

La méthode a été développée en employant le résorcinol comme étalon interne. Différents tests ont conduit au développement d'une méthode d'extraction liquide-liquide réalisée à pH 4.0 en présence d'acétate d'éthyle comme solvant, et en saturant la phase aqueuse en NaCl. L'extraction est réalisée pendant 30 min sous agitation circulaire. La phase organique est prélevée et le solvant acétate d'éthyle est évaporé sous un flux d'azote N₂ à 30°C. L'extrait sec obtenu après évaporation complète est alors repris dans un volume déterminé d'un mélange 50 % -50 % éthanol/H₂0.

Le rendement d'extraction a été mesuré par spectroscopie de masse après chromatographie haute pression sur une colonne C18 (dimensions : 100mm*2.1 mm ; granulométrie :1.7 µm) en employant un gradient 0.03% acide méthanoïque (HCOOH)/ acétonitrile (ACN).

Les rendements d'extraction ont été déterminés et mesurés en utilisant des solutions de phloroglucinol et résorcinol préparées dans le milieu de culture utilisé pour la croissance des levures. Les concentrations de phloroglucinol correspondent aux points bas (20 µg.mL⁻¹) et haut (200 µg.mL⁻¹) de la gamme de dosage. La concentration du résorcinol correspond à la concentration ajoutée en tant qu'étalon interne lors des dosages (200 µg.mL⁻¹). Les résultats sont présentés dans le Tableau 4.

**Tableau 4 : Rendement d'extraction du phloroglucinol (20 et 200 µg.mL⁻¹) et du résorcinol (200 µg.mL⁻¹), extraits par l'acétate d'éthyle, selon la méthode décrite.**

| **Produit** | **Phloroglucinol** | | **Résorcinol (EI)** |
|---|---|---|---|
| **Concentration de phloroglucinol ou de résorcinol dans le milieu de culture (µg.mL⁻¹)** | 20 | 200 | 200 |
| **RDT (%)** | 76 | 89 | 82 |

### 1.2.2. Développement d'une méthode d'analyse UPLC/UV et UPLC/Mass

Une méthode d'analyse par chromatographie UPLC et mesure d'absorbance en UV (rayonnement ultraviolet) a été développée. L'extrait est chromatographié sur une colonne propyl-pentafluorophenyl (PFP) de dimension 100*2.1 mm 1.8 µm selon un gradient de 0.1% HCOOH/ACN - 0.1% HCOOH. Le phloroglucinol est détecté en UV à 230 nm. Une méthode UPLC couplée à un spectromètre de masse (UPLC/Mass) a également été développée.

La quantification est réalisée en employant une gamme de 20 à 200 µg.ml⁻¹ de phloroglucinol dilué dans du milieu de culture de levure (Yeast Extract 1%, BactoPeptone 2 %) en présence d'une quantité fixe de résorcinol, employé comme témoin interne. La quantité de phloroglucinol est déterminée en calculant les rapports de surface des pics de chromatographie phloroglucinol / résorcinol.

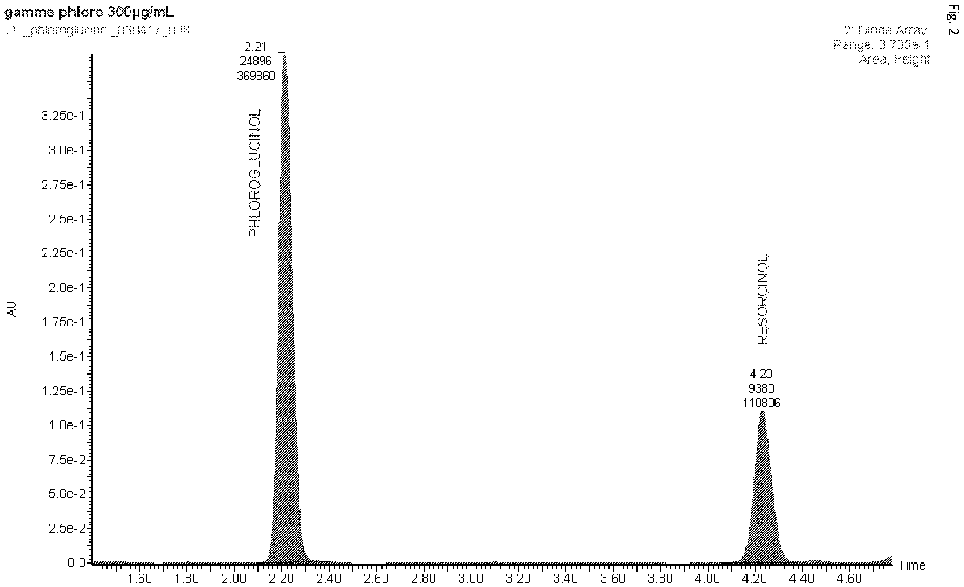

La Figure 2 présente un exemple de pics de chromatographie de phloroglucinol / résorcinol obtenus sur colonne PFP.

Cette méthode de dosage permet donc de mesurer qualitativement et quantitativement, de manière fiable, le phloroglucinol présent dans un échantillon.

La méthode UPLC couplée à un spectromètre de masse (UPLC/mass) permet de doser les échantillons contenant une concentration de phloroglucinol allant de 2 à 50 µg/mL.

### Exemple 2 : Reconstitution d'une forme active en tant que phloroglucinol synthase de l'enzyme PHLD.Aa d'Aureococcus anophagefferens

Les premiers résultats des tests d'activité réalisés avec la séquence *d'Aureococcus anophagefferens,* telle qu'annotée dans les bases de données (notée PHLD.Aa), identifiée dans l'exemple 1 comme une polyketide synthase de type III putative, n'avait pas conduit à la production de phloroglucinol (voir l'exemple 3 ci-dessous).

Une analyse comparative de la séquence du gène PHLD.Aa correspondant a été réalisée afin de comprendre cette absence d'activité. Cette séquence a été décrite suite à l'annotation du génome de l'algue *Aureococcus anophagefferens* (version AURANDRAFT_25482 de la séquence génomique).

L'analyse comparative de la séquence a permis de mettre en évidence de manière surprenante que la séquence protéique de l'enzyme PHLD.Aa telle qu'annotée dans les banques de données est significativement plus courte dans sa partie amino-terminale que les séquences des 3 autres enzymes ou candidates. Ce résultat suggérait donc une erreur d'annotation du génome.

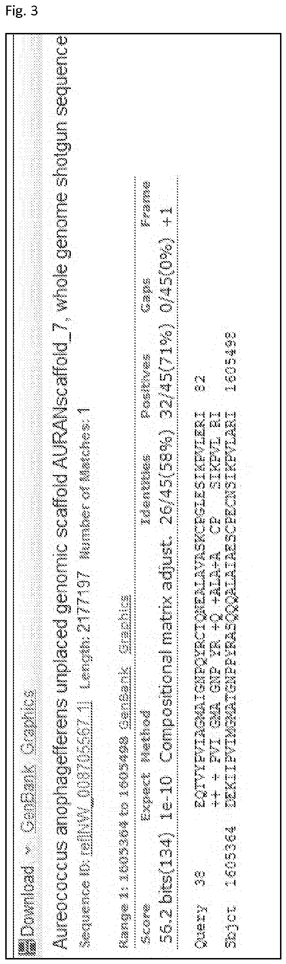

Une séquence nucléique codant pour une séquence protéique ressemblant à la partie amino-terminale du gène candidat PHLD.Sbi de *Sargassum binderi* a donc été recherchée dans la version AURANDRAFT-25482 du génome d'A. *anophagefferens.* Pour cela, la séquence protéique de la partie amino-terminale de la protéine PHLD.Sbi a été alignée avec la séquence résultant de la traduction des 6 phases des contigs de séquences du génome d'A. *anophagefferens,* en utilisant le logiciel Blast. Le résultat de cette recherche est présenté sur la Figure 3.

La Figure 3 montre que le génome d'A. *anophagefferens* contient bien une séquence nucléotidique codant une séquence peptidique très proche de la partie amino-terminale des gènes PKS1.Es et PHLD.Sbi.

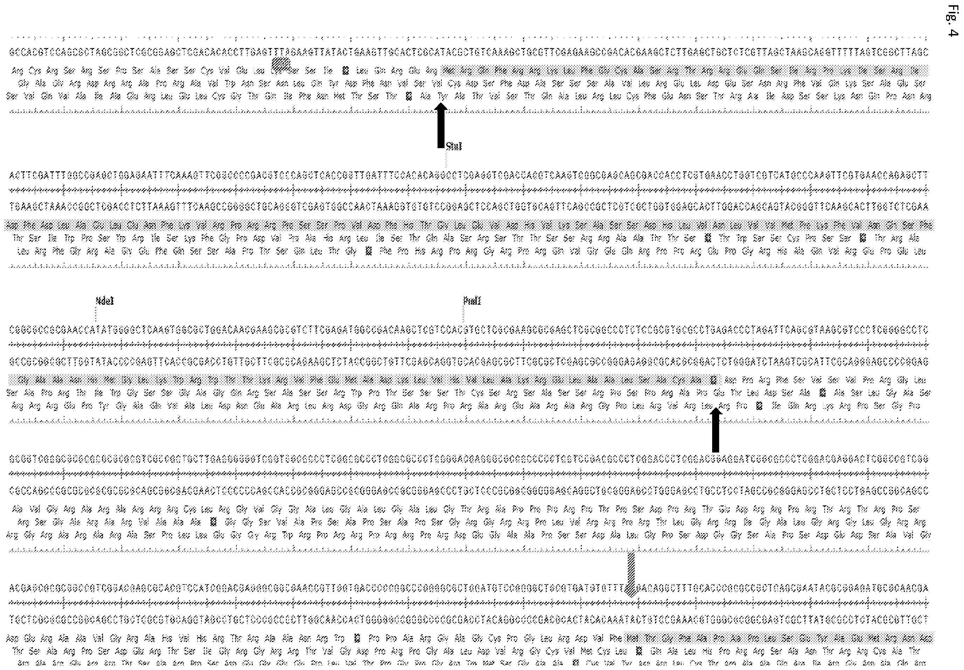

La Figure 4 montre la localisation de la séquence génomique d'A. *anophagefferens* codant une séquence peptidique homologue à la partie amino-terminale de PHLD.Sbi (séquence surlignée en gris clair encadrée par 2 flèches noires) située en 5' de la séquence du gène PHLD.Aa (flèche en gris foncé) tel qu'annoté dans les banques de données génomiques. Ainsi, cette séquence est positionnée en amont de la partie 5' du gène PHLD.Aa dans le génome d'A. *anophagefferens* tel qu'annoté et est séparée de cette dernière par une séquence nucléotidique non codante d'environ 300 paires de bases.

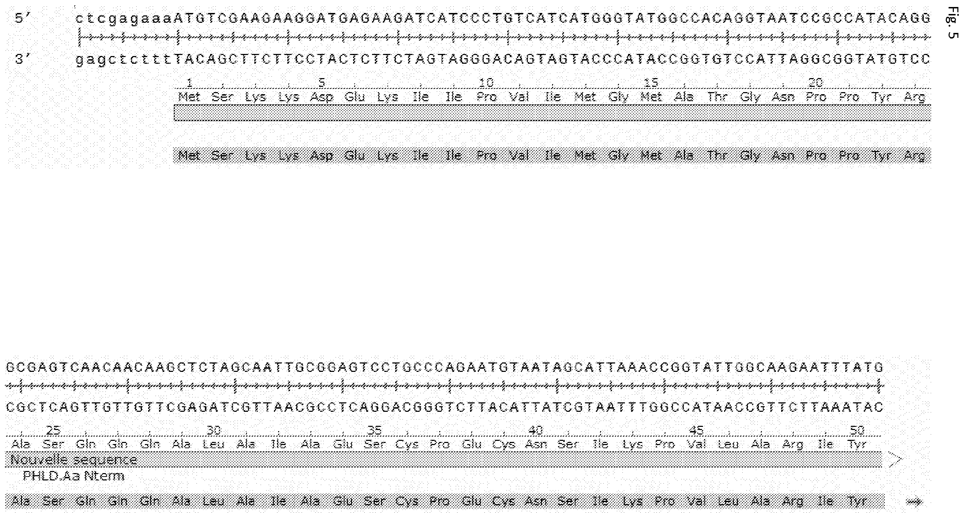

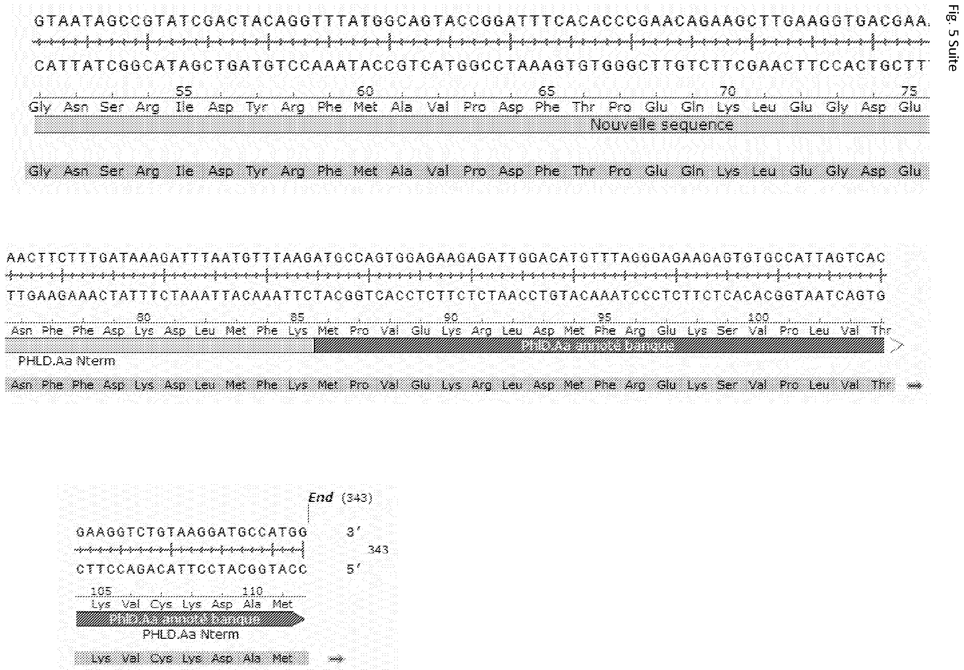

La probabilité que cette configuration soit maintenue sans signification dans le génome d'A. *anophagefferens* est infime. Il était donc vraisemblable que cette séquence de 300 paires de base corresponde soit à une séquence intronique, soit à une erreur d'assemblage de la séquence génomique dans les banques de données. Ces résultats suggéraient que la séquence de l'enzyme PHLD.Aa est en réalité constituée de l'association de ces deux séquences peptidiques qui sont codées par la séquence reconstituée montrée à la Figure 5.

La séquence complète de l'enzyme d'A. *anophagefferens* a donc été reconstituée par les Inventeurs en associant ces deux séquences pour former la protéine dénommée PHLD-1.Aa. La jonction des deux séquences a conduit à l'établissement de la séquence peptidique PHLD-1.Aa présentant une partie amino-terminale proche de celle de PHLD.Sbi (Figure 5).

L'alignement de la séquence nucléotidique codant l'enzyme reconstituée PHLD-1.Aa avec les séquences des autres gènes candidats PHLD a confirmé que la séquence de PHLD-1.Aa est plus proche des autres candidats identifiés dans l'exemple 1 (données non montrées).

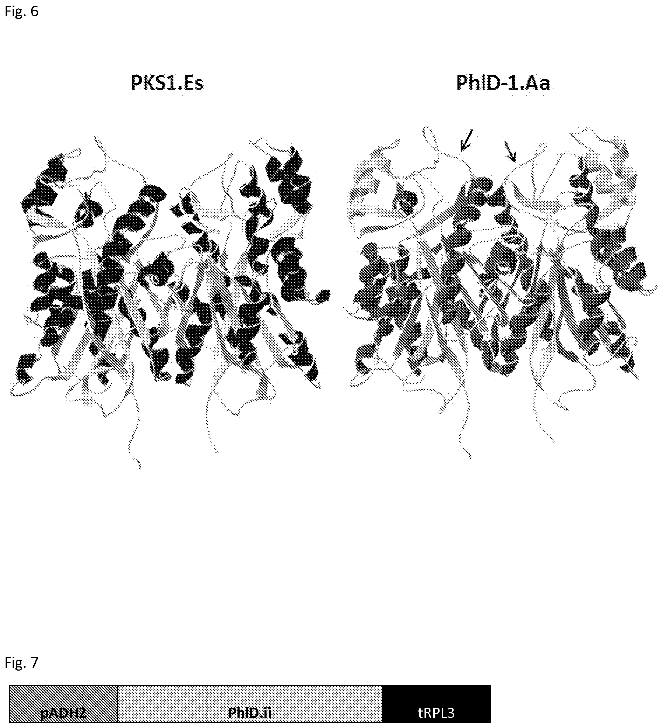

La structure tridimensionnelle de l'enzyme reconstituée PHLD-1.Aa a été modélisée, et a été comparée à celle de l'enzyme PKS1 d'*E. siliculosus* (établie par Meslet-Cladière et al., 2013). Il apparait clairement que l'ajout de la séquence amino-terminale ici identifiée permet une bien meilleure juxtaposition des structures de ces deux enzymes, dont les formes dimériques sont représentées par la Figure 6.

L'enzyme PHLD-1.Aa a été clonée sous contrôle du promoteur ADH2 et la construction génique résultante a été intégrée au locus URA3 de la souche de levure W303 (intégration multicopies). Les transformants obtenus ont été mis en culture en présence d'éthanol 20 g.L⁻¹ comme source de carbone et la production de phloroglucinol a été mesurée après 48 heures de culture à 30°C. Les résultats obtenus montrent que les cellules de levure exprimant la forme reconstituée PHLD-1.Aa produisaient des quantités significatives de phloroglucinol (de l'ordre de 200 mg.L⁻¹, voir l'Exemple 3 ci-dessous), à la différence des souches de levures exprimant la forme « tronquée » PHLD.Aa issue du génome annoté. Ces résultats confirment donc que la séquence PHLD.Aa présente dans les bases de données est incomplète et que l'identification par les Inventeurs d'une partie amino-terminale dans la séquence génomique a permis d'établir que la forme PHLD-1.Aa d'A. *anophagefferens* constitue une nouvelle phloroglucinol synthase.

### Exemple 3 : Identification de nouvelles phloroglucinol synthases fonctionnelles

### 3.1. Expression des gènes candidats dans la levure Saccharomyces cerevisiae sous forme multicopies

Les 2 gènes candidats PHLD-1.Aa et PHLD.Sbi, ainsi que les gènes PHLD.Pf et PKS1.Es codant les deux phloroglucinol synthases identifiées à ce jour et utilisées comme contrôles (voir Tableau 1 ; lignes grisées) et le gène candidat PHLD.Aa, ont été synthétisés en adaptant les codons utilisés pour une expression optimale dans la levure S. *cerevisiae.*

Cette adaptation des codons a été réalisée afin d'optimiser l'expression de ces différents gènes dans les cellules de levure (ces 5 gènes synthétiques codent des protéines strictement identiques aux protéines ou protéines putatives exprimées par les organismes d'origine). Chacun de ces gènes a été placé sous le contrôle du même promoteur de levure ADH2 (pADH2) qui permet leur expression notamment lorsque le milieu de culture contient de l'éthanol comme source de carbone. Le terminateur de transcription du gène de levure RPL3 (tRPL3) a été placé en aval de chacun des 5 gènes placés sous le contrôle du promoteur ADH2.

La Figure 7 montre un exemple d'unité génique ainsi construite pour un candidat ou un contrôle donné (PHLD.ii).

Les différentes unités géniques ainsi construites ont été intégrées de manière indépendante au locus URA3 du génome d'une souche sauvage de la levure S. *cerevisiae.* La souche de type sauvage utilisée est la souche commerciale W303 (génotype : MAT-a, his3, leu2, trp1, ura3, ade-). La technique d'intégration employée permet l'intégration d'un nombre variable de copies de chaque unité génique. Pour chaque construction, le nombre de copies d'unités géniques intégrées a été déterminé par PCR quantitative selon la méthode classique Taqman. Les souches de levure exprimant différents nombres de copies de chacun des 3 gènes candidats PHLD.ii ou des gènes contrôles PHLD.Pf et PKS1.Es décrits ci-dessus, ont été cultivées en présence de 20 g.L⁻¹ d'éthanol comme source de carbone pendant 48 heures à 30 °C. Les 5 souches de levures obtenues de manière indépendante après transformation et intégration au locus *URA3* des unités géniques décrites ci-dessus ont ainsi été analysées pour leur capacité à produire du phloroglucinol. La souche parentale sauvage W303 a été cultivée dans les mêmes conditions et utilisée à titre de témoin.

Les densités optiques (DO) des différentes cultures ont été mesurées à 600 nm (DO₆₀₀), indiquant ainsi le niveau de croissance de chaque souche (Figures 8A et B).

La capacité des différentes souches de levures exprimant les différents gènes PHLD.ii, sous le contrôle du promoteur ADH2, à synthétiser du phloroglucinol, a été testée en utilisant la méthode d'extraction et de dosage mise au point telle que décrite au paragraphe 1.2 ci-dessus. Le niveau de production de phloroglucinol (en mg.L⁻¹) a été mesuré dans le milieu de culture (Figures 8A et C).

La Figure 8 montre que l'ensemble des souches exprimant des nombres différents de copies des gènes PHLD.Sbi, PHLD-1.Aa, et PKS1.Es, produit une quantité significative de phloroglucinol qui est excrété dans le milieu de culture (Fig. 8A, C et D). Ces résultats indiquent donc que chacun de ces 3 gènes exprime une phloroglucinol synthase active chez la levure.

De manière surprenante, les résultats montrent que l'enzyme candidate PHLD.Aa ne présente pas d'activité de phloroglucinol synthase dans les conditions testées. Les Inventeurs ont pu mettre en évidence le fait que la protéine PHLD.Aa telle qu'annotée dans les banques de données est significativement plus courte dans sa partie amino-terminale que les autres PHLD (voir l'exemple 2). L'enzyme candidate PHLD-1.Aa présente quant à elle une activité de phloroglucinol synthase significative dans les conditions testées. Ces résultats montrent que le gène reconstitué et ré-annoté selon l'exemple 2 (PHLD-1.Aa) produit une phloroglucinol synthase PHLD fonctionnelle chez la levure.

De plus, comme attendu, aucune production de phloroglucinol n'a été mesurée dans la souche parentale contrôle W303 (données non montrées).

Ainsi, cette étude fonctionnelle a permis d'identifier deux nouvelles phloroglucinol synthases, codées respectivement par les gènes PHLD.Sbi et PHLD-1.Aa. Cette étude révèle pour la première fois que les algues S. *binderi* et *A. anophagefferens* codent des phloroglucinol synthases fonctionnelles (PHLD.Sbi et PHLD-1.Aa). Cette étude révèle également pour la première fois que l'enzyme PKS1.ES est fonctionnelle lorsqu'elle est exprimée chez un système eucaryote hétérologue, la levure.

Cette étude révèle en outre de manière inattendue que PHLD.Pf ne code pas une phloroglucinol synthase fonctionnelle chez la levure. Ce résultat est surprenant car l'enzyme codée par PHLD.Pf présente une activité phloroglucinol synthase démontrée lorsqu'elle est exprimée chez *Escherichia coli* (Achkar et al., 2005).

### 3.2. Expression des gènes candidats dans la levure Saccharomyces cerevisiae sous forme d'une copie unique

La production de phloroglucinol par des souches n'ayant intégré qu'une seule copie des gènes candidats PHLD.ii sélectionnés et identifiées comme étant fonctionnels chez la levure (voir les résultats du paragraphe précédent et la Fig. 8) a également été évaluée. Le gène PKS1.Es codant la phloroglucinol synthase fonctionnelle chez la levure selon les résultats décrits au paragraphe 3.1 ci-dessus a été utilisé comme contrôle positif.

Chacun de ces gènes a été placé soit sous le contrôle du promoteur de levure ADH2 (pADH2), qui permet leur expression notamment lorsque le milieu de culture contient de l'éthanol comme source de carbone, soit sous contrôle du promoteur de levure CCW12 (pCCW12), qui permet leur expression, notamment lors de la glycolyse, lorsque le milieu de culture contient du glucose comme source de carbone. Le terminateur de transcription du gène de levure RPL3 (tRPL3) a été placé en aval de chacune des constructions.

Le détail des différentes constructions géniques réalisées est reporté en Figure 9.

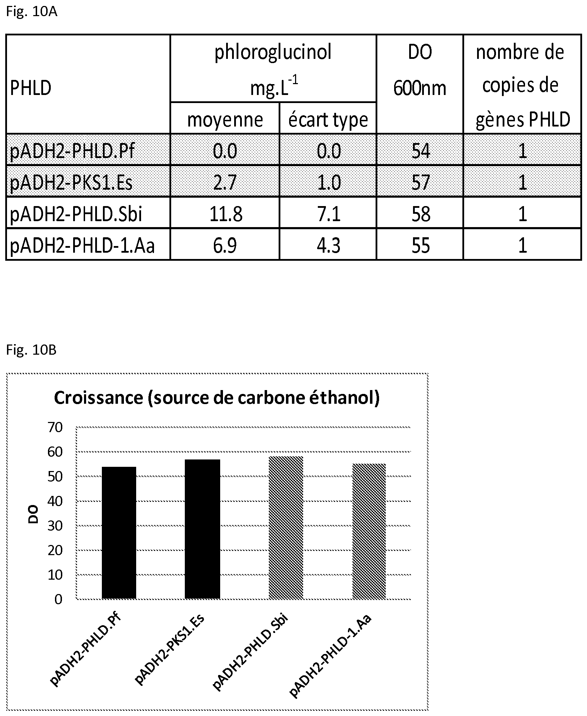

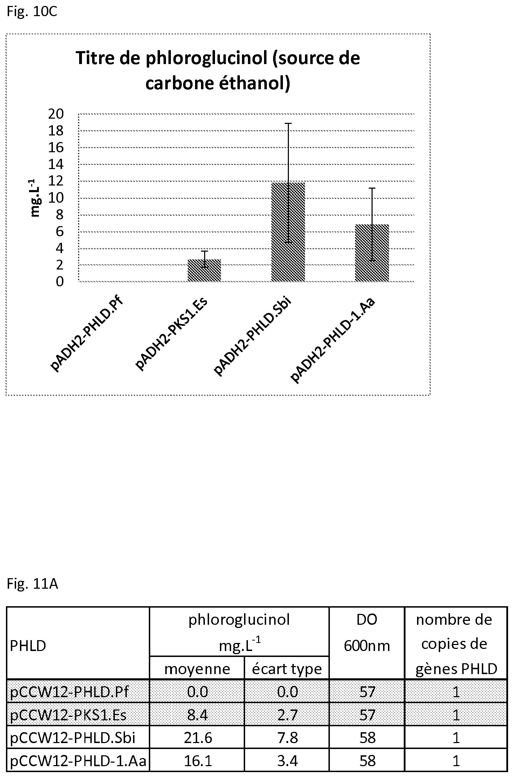

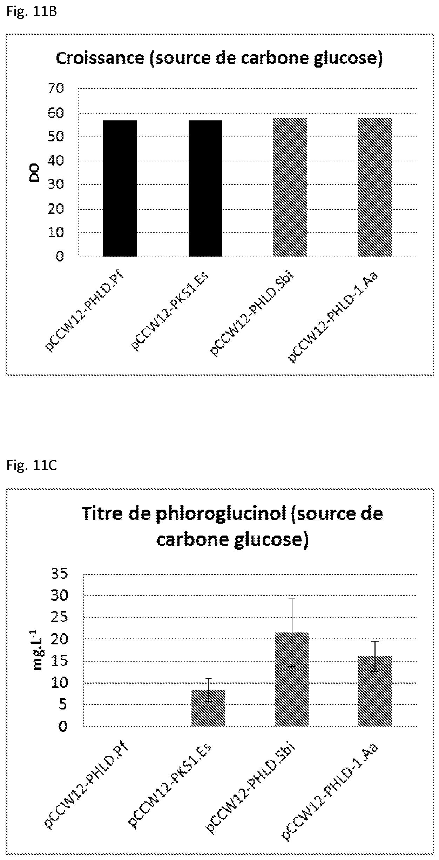

Les souches de levure exprimant une copie unique de PHLD ou PKS1 ont été cultivées en présence de 20 g.L⁻¹ d'éthanol comme source de carbone (constructions contrôlées par le pADH2) ou en présence de 20g. L⁻¹ de glucose (constructions contrôlées par le pCCW12) pendant 48 heures à 30 °C (Figures 10 et 11). Les souches ont été cultivées et analysées pour leur capacité à produire du phloroglucinol.

Les densités optiques des différentes cultures ont été mesurées à 600 nm (indiquant le niveau de croissance de chaque souche ; Figures 10A et 10B, Figures 11A et 11B) et la production de phloroglucinol (en mg.L⁻¹) a été mesurée dans le milieu de culture, pour 2 transformants indépendants pour chaque construction (Figures 10A et 10C, Figures 11A et 11C).

La Figure 10 montre que les souches exprimant une seule copie des gènes PHLD.Sbi et PHLD-1 .Aa sous le contrôle du promoteur ADH2 synthétisent une quantité mesurable et significative de phloroglucinol qui est excrétée dans le milieu de culture (Figures 10A et 10C).

Pour les cultures réalisées en présence de glucose comme source de carbone, les souches exprimant une seule copie des gènes PKS1.Es, PHLD.Sbi et PHLD-1.Aa sous le contrôle du promoteur CCW12 synthétisent toutes du phloroglucinol, mais en quantité plus élevée que pour les cultures réalisées en présence d'éthanol comme source de carbone (Figures 11A et 11C).

Les résultats obtenus ci-dessus confirment les résultats obtenus dans les souches contenant plusieurs copies des phloroglucinol synthases décrits dans le paragraphe 3.1 ci-dessus. Ainsi, l'expression des enzymes candidates PHLD.Sbi et PHLD-1.Aa, conduit à la production significative de phloroglucinol par les cellules de levure. Ces résultats montrent que ces gènes codent des phloroglucinol synthases et que ces phloroglucinol synthases sont actives dans des cellules de levure.

En outre, ces résultats montrent que les taux de production de phloroglucinol sont élevés même lorsqu'une seule copie du gène codant la phloroglucinol synthase est exprimée. En outre, l'activité mesurée dans les souches comprenant une seule copie est comparable à l'activité obtenue mesurée et divisée par le nombre de copies de chaque gène inséré (donc rapporté à 1 copie de chaque gène) dans les souches comportant les gènes PHLD.ii sous forme de multicopies.

Ces résultats sont les premières démonstrations de l'existence d'une activité phloroglucinol synthase chez les algues S. *binderi et A. anophagefferens.*

### 3.3. Observations complémentaires

L'étude réalisée par les Inventeurs et rapportée ici a permis d'identifier 2 nouvelles enzymes présentant une activité phloroglucinol synthase chez les algues (PHLD.Sbi et PHLD-1.Aa).

Les résultats démontrent également pour la première fois et de manière univoque qu'il est possible de synthétiser du phloroglucinol en cellules de levure.

Il est important de noter que le phloroglucinol synthétisé est sécrété à plus de 95 % dans le milieu de culture par les cellules de levure. Cette sécrétion particulièrement efficace est très favorable à la mise en oeuvre d'un procédé de bio-production du phloroglucinol.

Enfin, les enzymes identifiées par les Inventeurs sont originales en termes d'espèces d'origine et en termes de séquences protéiques.

En effet, il est montré pour la première fois que des algues appartenant aux genres *Sargassum* et *Aureococcus* codent des phloroglucinol synthase fonctionnelles. Cette découverte est d'autant plus surprenante que les précédentes tentatives d'identification de phloroglucinol synthase chez *Sargassum sp.* ont échoué (Baharum et al., 2011).

En outre, les séquences nucléiques et protéiques de l'enzyme PHLD-1.Aa d'A *anophagefferens* sont ici décrites pour la première fois.

### RÉFÉRENCES BIBLIOGRAPHIQUES

Achkar J et al., (2005) « Biosynthesis of phloroglucinol » J Am Chem Soc. 127:5332-5333*.*
Baharum H. et al., (2011) "Molecular Cloning, Modeling, and Site-Directed Mutagenesis of Type III Polyketide Synthase from Sargassum binderi" Mar Biotechnol. 13:845-856*.*
Meslet-Cladière L, Delage L, Leroux CJ, Goulitquer S, Leblanc C, Creis E, Gall EA, Stiger-Pouvreau V, Czjzek M, and Potin P. (2013) "Structure/function analysis of a type III polykétide synthase in the brown olga Ectocarpus siliculosus reveals a biochemical pathway in phlorotannin monomer biosynthesis. " Plant Cell. 25:3089-3103*.*
Zha W, Rubin-Pitel SB and Zhao H. (2006) "Characterization of the substrate specificity of PHLD, a type III polykétide synthase from Pseudomonas fluorescens. " J Biol Chem. 281:32036-32047*.*

## Revendications

1. Utilisation d'au moins un polypeptide choisi parmi les polyketide synthases de type III d'algues, comme phloroglucinol synthase, à l'exclusion des polyketide synthases de type III *d'Ectocarpus siliculosus* choisies parmi PKS1.Es, PKS2.Es, PKS3.Es ; ledit polypeptide comprenant au moins une séquence d'acides aminés ayant au moins 80% d'identité avec une séquence choisie parmi SEQ ID NO: 3 et SEQ ID NO: 1.

2. Utilisation selon la revendication 1, ledit polypeptide étant choisi parmi les polyketide synthases d'algues eucaryotes.

3. Utilisation selon la revendication 1 ou 2, ledit polypeptide étant choisi parmi les polyketide synthases de type III d'algues ochrophytes, de préférence d'algues *Marista,* de préférence encore d'algues appartenant à la classe des algues brunes ou à la classe des algues pélagophytes.

4. Utilisation selon l'une quelconque des revendications 1 à 3, ledit polypeptide comprenant au moins une séquence d'acides aminés ayant au moins85% d'identité avec une séquence choisie parmi SEQ ID NO: 3 et SEQ ID NO: 1.

5. Utilisation selon la revendication 3, lesdites algues ochrophytes étant choisies dans le groupe constitué par *Aureococcus sp.* et *Sargassum sp.,* en particulier *Aureococcus anophagefferens* et *Sargassum binderi.*

6. Utilisation selon l'une quelconque des revendications 1 à 5, ledit polypeptide comprenant au moins une séquence d'acides aminés ayant au moins 90% d'identité, de manière encore préférée au moins 91% d'identité, de manière encore plus préférée au moins 92% d'identité, de manière toujours plus préférée au moins 93% d'identité, de manière plus préférentielle encore au moins 94% d'identité, de manière davantage préférée au moins 95% d'identité, de manière encore préférée au moins 96% d'identité, de manière encore plus préférée au moins 97% d'identité, de manière toujours plus préférée au moins 98% d'identité, et de manière plus préférentielle encore au moins 99% d'identité, avec une séquence choisie parmi SEQ ID NO: 3 et SEQ ID NO: 1.

7. Utilisation selon l'une quelconque des revendications 1 à 6, ledit polypeptide comprenant au moins une séquence d'acides aminés choisie parmi SEQ ID NO: 3 et SEQ ID NO: 1.

8. Polypeptide isolé à activité de phloroglucinol synthase comprenant au moins une séquence d'acides aminés présentant au moins 80% d'identité avec la séquence SEQ ID NO: 3, de préférence au moins 85% d'identité, de préférence encore au moins 90% d'identité, de préférence encore au moins 91% d'identité, de préférence encore au moins 92% d'identité, de manière encore préférée au moins 93% d'identité, de manière encore plus préférée au moins 94% d'identité, de manière toujours plus préférée au moins 95% d'identité, de manière toujours plus préférée au moins 96% d'identité, de manière toujours plus préférée au moins 97% d'identité, de manière toujours plus préférée au moins 98% d'identité, et de manière plus préférentielle encore au moins 99% d'identité avec la séquence SEQ ID NO: 3.

9. Polypeptide isolé selon la revendication 8, comprenant au moins une séquence d'acides aminés de séquence SEQ ID NO: 3.

10. Molécule d'acides nucléiques isolée **caractérisée en ce qu'**elle code pour un polypeptide isolé selon l'une quelconque des revendications 8 à 9, ladite molécule d'acides nucléiques isolée ayant de préférence au moins 80% d'identité avec la séquence SEQ ID NO: 4, de préférence encore au moins 85% d'identité, de préférence encore au moins 90% d'identité, de préférence encore au moins 91% d'identité, de manière encore préférée au moins 92% d'identité, de manière encore plus préférée au moins 93% d'identité, de manière toujours plus préférée au moins 94% d'identité, de manière toujours plus préférée au moins 95% d'identité, de manière toujours plus préférée au moins 96% d'identité, de manière toujours plus préférée au moins 97% d'identité, de manière plus préférentielle encore au moins 98% d'identité, de manière plus préférentielle encore au moins 99% d'identité avec la SEQ ID NO: 4, et de manière encore plus préférée ayant pour séquence SEQ ID NO: 4.

11. Molécule d'acides nucléiques isolée comprenant au moins une séquence d'acides nucléiques codant un polypeptide tel que défini dans l'une quelconque des revendications 1 à 9 et comprenant en outre un promoteur contrôlant l'expression de ladite au moins une séquence d'acides nucléiques.

12. Vecteur comprenant au moins une molécule d'acides nucléiques selon l'une quelconque des revendications 10 à 11, ledit vecteur étant de préférence un plasmide.

13. Cellule hôte comprenant au moins une molécule d'acides nucléiques selon l'une quelconque des revendications 10 à 11, ou au moins un vecteur selon la revendication 12, la cellule hôte étant un microorganisme sélectionné parmi les bactéries, les levures, les champignons, et les cyanobactéries.

14. Méthode pour produire du phloroglucinol, comprenant les étapes consistant en :
(i) la mise en contact d'une cellule hôte exprimant le polypeptide à activité de phloroglucinol synthase tel que défini dans l'une quelconque des revendications 1 à 9, par exemple une cellule hôte selon la revendication 13, avec un substrat approprié ;
(ii) la culture *in vitro* de la cellule hôte de l'étape (i) dans des conditions permettant la croissance de ladite cellule hôte et/ou l'expression de la molécule d'acides nucléiques contenue dans ladite cellule hôte, de manière à produire du phloroglucinol ;
(iii) optionnellement la récupération du milieu de culture comprenant le phloroglucinol, obtenu après l'étape (ii) ; et
(iv) optionnellement, la purification du phloroglucinol à partir du milieu de culture de l'étape (iii).

15. Méthode pour produire du phloroglucinol, comprenant au moins les étapes consistant en :
(i) la mise en contact d'au moins un polypeptide tel que défini dans l'une quelconque des revendications 1 à 9 avec un substrat approprié ;
(ii) l'incubation du mélange issu de l'étape (i) dans des conditions adaptées pour produire du phloroglucinol;
(iii) optionnellement la récupération du milieu réactionnel comprenant le phloroglucinol, obtenu après l'étape (ii) ; et
(iv) optionnellement, la purification du phloroglucinol à partir du milieu réactionnel de l'étape (iii).

## Patentansprüche

1. Verwendung mindestens eines Polypeptids, das aus den Polyketid-Synthasen des Typs II von Algen, wie Phloroglucinol-Synthase, ausgewählt ist, mit Ausnahme der Polyketid-Synthasen des Typs III von Ectocarpus siliculosus, welche aus KS1.Es, PKS2.Es, PKS3.Es ausgewählt sind; wobei das Polypeptid mindestens eine Aminosäuresequenz umfasst, die eine Übereinstimmung von mindestens 80% mit einer Sequenz aufweist, welche aus SEQ ID Nr.: 3 und SEQ ID Nr.: 1 ausgewählt ist.

2. Verwendung nach Anspruch 1, wobei das Polypeptid aus den Polyketid-Synthasen eukaryotischer Algen ausgewählt ist.

3. Verwendung nach Anspruch 1 oder 2, wobei das Polypeptid aus den Polyketid-Synthasen des Typs III von Heterokontophyten-Algen, vorzugsweise von Marista-Algen, wiederum bevorzugt von Algen ausgewählt ist, welcher der Klasse der Braunalgen oder der Klasse der Pelagophyten-Algen angehören.

4. Verwendung nach einem beliebigen der Ansprüche 1 bis 3, wobei das Polypeptid mindestens eine Aminosäuresequenz umfasst, die eine Übereinstimmung von mindestens 85 % mit einer Sequenz aufweist, die aus SEQ ID Nr.: 3 und SEQ ID Nr: 1 ausgewählt ist.

5. Verwendung nach Anspruch 3, wobei die Heterokontophyten-Algen aus der Gruppe ausgewählt sind, die aus *Aureococcus sp.* und *Sargassum sp.,* insbesondere *Aureococcus anophagefferens* und *Sargassum binderi* besteht.

6. Verwendung nach einem beliebigen der Ansprüche 1 bis 5, wobei das Polypeptid mindestens eine Aminosäuresequenz umfasst, die eine Übereinstimmung von mindestens 90 %, auf stärker bevorzugte Weise von mindestens 91%, auf noch stärker bevorzugte Weise von mindestens 92%, auf wiederum stärker bevorzugte Weise von mindestens 93%, auf noch stärker bevorzugte Weise von mindestens 94%, auf stärker bevorzugte Weise von mindestens 95%, auf stärker bevorzugte Weise von mindestens 96%, auf noch stärker bevorzugte Weise von mindestens 97%, auf wiederum stärker bevorzugte Weise von mindestens 98% und auf noch stärker bevorzugte Weise von mindestens 99% mit einer Sequenz aufweist, die aus SEQ ID Nr.: 3 und SEQ ID Nr.: 1 ausgewählt ist.

7. Verwendung nach einem beliebigen der Ansprüche 1 bis 6, wobei das Polypeptid mindestens eine Aminosäuresequenz umfasst, die aus SEQ ID Nr.: 3 und SEQ ID Nr.: 1 ausgewählt ist.

8. Isoliertes Polypeptid mit Phloroglucinol-Synthase-Aktivität, das mindestens Aminosäuresequenz umfasst, die eine Übereinstimmung von mindestens 80 % mit der SEQ ID Nr.: 3, vorzugsweise eine Übereinstimmung von mindestens 85%, stärker bevorzugt eine Übereinstimmung von mindestens 90%, stärker bevorzugt eine Übereinstimmung von mindestens 91, stärker bevorzugt eine Übereinstimmung von mindestens 92%, auf noch stärker bevorzugte Weise eine Übereinstimmung von mindestens 93%, auf noch stärker bevorzugte Weise eine Übereinstimmung von mindestens 94%, auf wiederum stärker bevorzugte Weise eine Übereinstimmung von mindestens 95%, auf wiederum stärker bevorzugte Weise eine Übereinstimmung von mindestens 96%, auf wiederum stärker bevorzugte Weise eine Übereinstimmung von mindestens 97%, auf wiederum stärker bevorzugte Weise eine Übereinstimmung von mindestens 98% und auf noch stärker bevorzugte Weise von mindestens 99% mit der SEQ ID Nr.: 3 aufweist.

9. Isoliertes Polypeptid nach Anspruch 8, das mindestens eine Aminosäuresequenz mit der Sequenz SEQ ID Nr.: 3 umfasst.

10. Isoliertes Nukleinsäuremolekül, **dadurch gekennzeichnet, dass** es für ein isoliertes Polypeptid nach einem beliebigen der Ansprüche 8 bis 9 codiert, wobei das isolierte Nukleinsäuremolekül vorzugsweise eine Übereinstimmung von mindestens 80% mit der Sequenz SEQ ID Nr.: 4, stärker bevorzugt eine Übereinstimmung von mindestens 85%, stärker bevorzugt eine Übereinstimmung von mindestens 90%, stärker bevorzugt eine Übereinstimmung von mindestens 91%, auf noch stärker bevorzugte Weise eine Übereinstimmung von mindestens 92%, auf noch stärker bevorzugte Weise eine Übereinstimmung von mindestens 93%, auf wiederum stärker bevorzugte Weise eine Übereinstimmung von mindestens 94%, auf wiederum stärker bevorzugte Weise eine Übereinstimmung von mindestens 95%, auf wiederum stärker bevorzugte Weise eine Übereinstimmung von mindestens 96%, auf wiederum stärker bevorzugte Weise eine Übereinstimmung von mindestens 97%, auf noch stärker bevorzugte Weise eine Übereinstimmung von mindestens 98% und auf stärker bevorzugte Weise von mindestens 99% mit der SEQ ID Nr.: 4 aufweist, und sie auf noch stärker bevorzugte Weise SEQ ID Nr.: 4 aufweist.

11. Isoliertes Nukleinsäuremolekül, das mindestens eine Nukleinsäuresequenz umfasst, welche für ein Polypeptid gemäß der Begriffsbestimmung in einem beliebigen der Ansprüche 1 bis 9 codiert und darüber hinaus einen Promotor umfasst, welche die Expression der mindestens einen Nukleinsäuresequenz steuert.

12. Vektor, der mindestens ein Nukleinsäuremolekül nach einem beliebigen der Ansprüche 10 bis 11 umfasst, wobei es sich bei dem Vektor vorzugsweise um ein Plasmid handelt.

13. Wirtszelle, die mindestens ein Nukleinsäuremolekül nach einem beliebigen der Ansprüche 10 bis 11 oder mindestens einen Vektor nach Anspruch 12 umfasst, wobei es sich bei der Wirtszelle um eine Mikroorganismus handelt, der aus den Bakterien, den Hefen, den Pilzen und den Cyanobakterien ausgewählt ist.

14. Verfahren zur Herstellung von Phloroglucinol, das die folgenden Schritte umfasst:
(i) Inkontaktbringen einer Wirtszelle, welche das Polypeptid mit Phloroglucinol-Aktivität gemäß der Begriffsbestimmung in einem beliebigen der Ansprüche 1 bis 9 umfasst, beispielsweise einer Wirtszelle nach Anspruch 13, mit einem geeigneten Substrat;
(ii) Anzüchten der Wirtszelle des Schrittes (i) *in vitro* unter Bedingungen, welche das Wachstum der Wirtszelle und/oder die Expression des Nukleinsäuremoleküls ermöglichen, welches in der Wirtszelle enthalten ist, um auf diese Weise Phloroglucinol zu produzieren;
(iii) möglicherweise Gewinnen des Kulturmediums, welches das Phloroglucinol umfasst, wie es nach dem Schritt (ii) erhalten wurde; und
(iv) möglicherweise Aufreinigen des Phloroglucinols ausgehend von dem Kulturmedium des Schrittes (iii).

15. Verfahren zur Herstellung von Phloroglucinol, das zumindest die folgenden Schritt umfasst:
(i) Inkontaktbringen mindestens eines Polypeptids gemäß der Begriffsbestimmung in einem beliebigen der Ansprüche 1 bis 9 mit einem geeigneten Substrat;
(ii) Inkubieren der Mischung aus dem Schritt (i) unter Bedingungen, die zur Produktion von Phloroglucinol geeignet sind;
(iii) möglicherweise Gewinnen des Reaktionsmilieus, welches das Phloroglucinol umfasst, wie es nach dem Schritt (ii) erhalten wurde; und
(iv) möglicherweise Aufreinigen des Phloroglucinols ausgehend von dem Reaktionsmilieu des Schrittes (iii).

## Claims

1. Use of at least one polypeptide chosen from type III polyketide synthases of algae, such as phloroglucinol synthase, with the exclusion of the type III polyketide synthases of *Ectocarpus siliculosus* chosen from PKS1.Es, PKS2.Es, PKS3.Es; said polypeptide comprising at least one amino acid sequence having at least 80% identity with a sequence chosen from SEQ ID NO: 3 and SEQ ID NO: 1.

2. Use according to Claim 1, said polypeptide being chosen from polyketide synthases of eukaryotic algae.

3. Use according to Claim 1 or 2, said polypeptide being chosen from type III polyketide synthases of ochrophyte algae, preferably of *Marista* algae, and more preferably of algae belonging to the class of brown algae or to the class of pelagophyte algae.

4. Use according to any one of Claims 1 to 3, said polypeptide comprising at least one amino acid sequence having at least 85% identity with a sequence chosen from SEQ ID NO: 3 and SEQ ID NO: 1.

5. Use according to Claim 3, said ochrophyte algae being chosen from the group consisting of *Aureococcus sp.* and *Sargassum sp.,* in particular *Aureococcus anophagefferens* and *Sargassum binderi.*

6. Use according to any one of Claims 1 to 5, said polypeptide comprising at least one amino acid sequence having at least 90% identity, more preferably at least 91% identity, even more preferably at least 92% identity, more preferably still at least 93% identity, even more preferentially at least 94% identity, further preferably at least 95% identity, more preferably at least 96% identity, even more preferably at least 97% identity, more preferably still at least 98% identity, and even more preferentially at least 99% identity, with a sequence chosen from SEQ ID NO: 3 and SEQ ID NO: 1.

7. Use according to any one of Claims 1 to 6, said polypeptide comprising at least one amino acid sequence chosen from SEQ ID NO: 3 and SEQ ID NO: 1.

8. Isolated polypeptide with phloroglucinol synthase activity, comprising at least one amino acid sequence having at least 80% identity with the sequence SEQ ID NO: 3, preferably at least 85% identity, more preferably at least 90% identity, more preferably at least 91% identity, more preferably at least 92% identity, more preferably at least 93% identity, even more preferably at least 94% identity, more preferably still at least 95% identity, more preferably still at least 96% identity, more preferably still at least 97% identity, more preferably still at least 98% identity, and even more preferentially at least 99% identity with the sequence SEQ ID NO: 3.

9. Isolated polypeptide according to Claim 8, comprising at least one amino acid sequence of sequence SEQ ID NO: 3.

10. Isolated nucleic acid molecule, **characterized in that** it codes for an isolated polypeptide according to any one of Claims 8 to 9, said isolated nucleic acid molecule preferably having at least 80% identity with the sequence SEQ ID NO: 4, more preferably at least 85% identity, more preferably at least 90% identity, more preferably at least 91% identity, more preferably at least 92% identity, even more preferably at least 93% identity, more preferably still at least 94% identity, more preferably still at least 95% identity, more preferably still at least 96% identity, more preferably still at least 97% identity, even more preferentially at least 98% identity, and even more preferentially at least 99% identity with the sequence SEQ ID NO: 4, and even more preferably having, as sequence, SEQ ID NO: 4.

11. Isolated nucleic acid molecule comprising at least one nucleic acid sequence coding for a polypeptide as defined in any one of Claims 1 to 9 and additionally comprising a promoter controlling the expression of said at least one nucleic acid sequence.

12. Vector comprising at least one nucleic acid molecule according to any one of Claims 10 to 11, said vector preferably being a plasmid.

13. Host cell comprising at least one nucleic acid molecule according to any one of Claims 10 to 11, or at least one vector according to Claim 12, the host cell being a microorganism selected from bacteria, yeasts, fungi and cyanobacteria.

14. Method for producing phloroglucinol, comprising the steps consisting of:
(i) contacting a host cell expressing the polypeptide with phloroglucinol synthase activity as defined in any one of Claims 1 to 9, for example a host cell according to Claim 13, with an appropriate substrate;
(ii) culturing the host cell of step (i) *in vitro* under conditions permitting the growth of said host cell and/or the expression of the nucleic acid molecule contained in said host cell, so as to produce phloroglucinol;
(iii) optionally, recovering the culture medium comprising the phloroglucinol, obtained after step (ii); and
(iv) optionally, purifying the phloroglucinol from the culture medium of step (iii).

15. Method for producing phloroglucinol, comprising at least the steps consisting of:
(i) contacting at least one polypeptide as defined in any one of Claims 1 to 9 with an appropriate substrate;
(ii) incubating the mixture obtained from step (i) under conditions suitable for producing phloroglucinol;
(iii) optionally, recovering the reaction medium comprising the phloroglucinol, obtained after step (ii); and
(iv) optionally, purifying the phloroglucinol from the reaction medium of step (iii).
